# EUROPÄISCHE PATENTSCHRIFT

(11) **EP 3 913 039 B1**
(45) Veröffentlichungstag und Bekanntmachung des Hinweises auf die Patenterteilung: **31.01.2024**
(21) Anmeldenummer: 21185857.6
(22) Anmeldetag: 11.04.2018
(51) Int. Cl.: C10M 105/40, C10M 105/42

(54) **MISCHUNG AUS ESTERVERBINDUNGEN**
BLEND OF ESTER COMPOUNDS
MÉLANGE DE COMPOSÉS ESTER

(30) Priorität: 13.04.2017 DE 102017003647; 10.04.2018 DE 102018002891
(43) Veröffentlichungstag der Anmeldung: 24.11.2021
(62) Teilanmeldung aus: 18726045.0
(73) Patentinhaber: Klüber Lubrication München SE & Co. KG, 81379 München (DE); Universität Bielefeld, 33615 Bielefeld (DE)
(72) Erfinder: BETKE, Tobias, 33615 Bielefeld (DE); PLASS, Carmen, 33615 Bielefeld (DE); GRÖGER, Harald, 33615 Bielefeld (DE); LODERER, Dirk, 81379 München (DE); SEEMEYER, Stefan, 81379 München (DE); KILTHAU, Thomas, 81379 München (DE); MA, Ling, 81379 München (DE)
(74) Vertreter: Hering, Hartmut

(56) Entgegenhaltungen:
- EP-A1- 1 051 465
- EP-B1- 1 051 465
- US-A1- 2006 276 609
- US-A1- 2010 249 357
- US-A1- 2014 252 281

## Beschreibung

Die Erfindung betrifft neue Esterverbindungen der allgemeinen Formeln **(Ia), (Ib), (Ic)**, wie in den Ansprüchen definiert.

Esterverbindungen sind in den letzten Jahren immer häufiger in Schmiermittelzubereitungen eingesetzt worden. Da die Estereinheiten in den Esterverbindungen in natürlichen Ölen wie auch in synthetischen Esterölen hydrolyseanfällig sind, besteht ein großer Bedarf an Esterverbindungen, die den hohen Anforderungen für den Einsatz in Schmiermitteln genügen. Bei den bekannten Esterverbindungen wird bei der Anwendung des Schmiermittels in Gegenwart von Wasser der Ester in die Fettsäure und den Alkohol gespalten. Diese Reaktion kann beispielsweise durch Säuren, Basen oder durch Kupfer katalysiert werden. Das hat die Zerstörung der Moleküle zur Folge, wodurch die Schmierstoffe an Schmierwirkung verlieren.

Des weiteren sind herkömmliche Schmierstoffe beispielsweise basierend auf nativen Estern für Hochtemperaturanwendungen nicht geeignet, da sie bei hohen Temperaturen durch Oxidations- und/oder thermische Zersetzungsvorgänge sowie durch Polymerisationen zerstört werden können und dadurch ihre schmierenden Eigenschaften und Wirkungen stark eingeschränkt werden. Bei Zersetzungsreaktionen wird der Schmierstoff in niedermolekulare flüchtige Komponenten gespalten. Das Verdampfen dieser flüchtigen Komponenten führt zu unerwünschten Viskositätsänderungen, Ölverlust und zur übermäßigen Dampfbildung. Hieraus resultiert ebenfalls ein Verlust der Schmierwirkung. Auch durch Polymerisation verlieren die Schmierstoffe aufgrund der Bildung unlöslicher Polymerisationsprodukte ihre Schmierwirkung. Diese Verschmutzungen müssen entfernt werden, wodurch sich die Wartungsarbeiten erhöhen. Es werden darüber hinaus chemische Abfallstoffe produziert, die aufwendig entsorgt werden müssen. Aufgrund der vermehrten Reinigungs- und Wartungsarbeiten erhöhen sich die Ausfallzeiten der zu schmierenden Vorrichtungen. Insgesamt führt die Verwendung von ungeeigneten Schmierstoffen bei Hochtemperaturanwendungen zu höheren Kosten, da die Arbeitsgeräte verschmutzen und ein höherer Bedarf an Schmierstoffen besteht. Darüber hinaus sinkt die Produktqualität.

Um den vielfältigen Anforderungen gerecht zu werden, müssen Schmierstoffe unter anderem eine hohe Stabilität, niedrige Reibungsbeiwerte und hohe Verschleißfestigkeiten aufweisen. Hohe Temperaturen treten oftmals bei der Verwendung in Ketten, Wälz- und Gleitlagern, in der Fahrzeugtechnik, der Fördertechnik, dem Maschinenbau, der Bürotechnik sowie in industriellen Anlagen und Maschinen, aber auch in den Bereichen der Haushaltsmaschinen und der Unterhaltungselektronik, auf. Hohe Verarbeitungstemperaturen treten beispielsweise oftmals bei der Lebensmittelverarbeitung auf, wie beim Kochen, Backen, Sieden, Rösten, Schmoren, Sterilisieren, Braten und Dämpfen. Bei diesen Vorgängen kommen diverse Arbeitsgeräte zum Einsatz. Zur Schmierung dieser Arbeitsgeräte sind hochtemperaturbeständige Schmierstoffe notwendig.

An die Basisöle zum Schmieren von Arbeitsgeräten für die Verarbeitung von Lebensmitteln werden besondere Anforderungen in Bezug auf ihre Umweltverträglichkeit und Toxizität gestellt. Grundsätzlich sollte ein lebensmittelverträglicher Schmierstoff H1 tauglich sein, wenn der Schmierstoff mittelbar oder unmittelbar mit Nahrungs-, Genuss- und Lebensmitteln in Kontakt kommen kann. Zu den bevorzugten Anwendungsbereichen in der Lebensmittelindustrie gehören Ketten in Backöfen und anderen Hochtemperaturanwendungen, sowie Transportgehänge, insbesondere Trolleys und deren Lager.

Diese Schmierstoffe unterliegen gesetzlichen Vorschriften, wie der Zertifizierung nach NSF/H1 oder NSF/H2.

Bei Anwendungen von Schmierstoffen im Marinebereich, die sich zumeist unterhalb der Wasserlinie befinden, besteht das Risiko, die Meeres- bzw. Gewässerumwelt durch Austritt von Schmierstoffen zu kontaminieren. Obwohl versucht wird, bei diesen Anwendungen die Wasserseite bestmöglich abzudichten, sind Schmierstoffverluste alltäglich. Laut einer Quelle der "United States Environmental Protection Agency" im Jahre 2011 verlieren unterschiedliche Schiffsbauten von weniger als einen Liter Schmierstoff bis hin zu 20 Liter pro Tag und Schiff. Eine gute biologische Abbaubarkeit des Schmierstoffes ist hierbei Voraussetzung für eine hohe Umweltverträglichkeit der Schmiermittel. Generell werden solche Anwendungen über gesetzliche Anforderungen oder Normen z.B. VGP, Eco-Label oder OSPAR geregelt.

Die bisher bekannten Schmierstoffe, können aber allen diesen Anforderungen nicht genügen.

Es wurde bereits vorgeschlagen, sogenannte Estolidverbindungen in Schmierstoffen einzusetzen. Diese Esterverbindungen, die in der Regel auf Pflanzenölen beruhen, werden gemäß S. C. Cermak et al., Industrial Crops and Products 2013, 46, 386-392, synthetisiert. Gemäß dieser Literatur sowie der Patentschrift EP 1 051 465 B1 werden dabei Verbindungen der in Abbildung 1 dargestellten allgemeinen Struktur **Lit-1** erhalten, wobei x und y gleich oder größer als 1, x+y gleich 10, n gleich oder größer 1, R gleich CHR₁R₂, R₁ und R₂ unabhängig voneinander ausgewählt aus Wasserstoff und gesättigten oder ungesättigten, verzweigten oder unverzweigten, substituierten oder unsubstituierten und C1- bis C36-Kohlenwasserstoffen, R₃ ein aus Ölsäure, Sterearinsäure oder anderen Fettsäuren resultierendes strukturelles Fragment sind und die bevorzugte Position der sekundären Esterverzweigung an der 9- oder 10-Position ist (entsprechend x=5 bzw. 6 und y=5 bzw. 4).

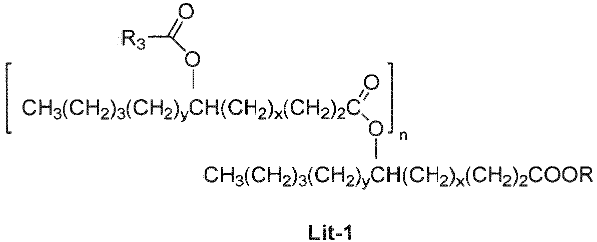

### Abbildung 1. Allgemeine Struktur der literaturbekannten Estolid-Verbindungen

Die Herstellung dieser sogenannten Estolid-Verbindungen vom Typ **Lit-1** erfolgt gemäß der oben genannten Literatur durch Addition einer Fettsäure an die C=C-Doppelbindung der nächsten Fettsäure, so dass entsprechende Dimere (n = 0) sowie darüber hinaus auch Tri-, Tetra-, Penta- und Hexamere (n = 1, 2, 3, 4) erhalten werden. Allerdings kann die dargestellte Struktur aus Perspektive der wissenschaftlichen Darstellung einer repititiven Einheit nicht richtig sein, da die Verknüpfung der repititiven Einheit daraus nicht hervorgeht. Nachteilig an diesem Verfahren sind die niedrige Selektivität (die entsprechend statt zu einer definierten Produktverbindung zu einem Gemisch aus Dimer und Oligomergemischen führt), die harschen Reaktionsbedingungen, die geringe Ausbeute sowie die aufwändige Trennung von Dimer/Oligomergemischen. Die beiden abschließenden Schritte bilden dann die Hydrierung der verbliebenen C=C-Doppelbindung sowie die Veresterung der verbleibenden Carbonsäuregruppe, wobei als Alkoholkomponente vorzugsweise 2-Ethylhexan-1-ol eingesetzt wird.

Die US 2014/252281 A1 offenbart eine Mischung aus zwei Esterverbindungen, die durch Umsetzung von Ölsäure durch Hydroformulierung und Hydrierung zu regioisomeren Verbindungen, beispielsweise 2-Ethylhexyl-10-methyloxydecanolystearat und 2-Ethylhexyl-9-methyloxydecanolystearat, erhalten werden. Diese Esterverbindungen werden als dielektrische Isolationsflüssigkeit für elektrische Apparate eingesetzt.

Der vorliegenden Erfindung lag daher die Aufgabe zu Grunde, neuartige Esterverbindungen bereitzustellen, die den oben genannten Anforderungen für die Verwendung in Schmiermitteln genügen und die aus einfachen und leicht zugänglichen, vorzugsweise nachwachsenden Rohstoffen als Ausgangsmaterialien auf nachhaltigen und umweltfreundlichen Wege herzustellen sind. Darüber hinaus sollte das Syntheseverfahren eine hohe Selektivität aufweisen, eine hohe Ausbeute erzielt werden und eine einfache Aufarbeitung möglich sein.

Diese Aufgabe wurde durch die Bereitstellung neuartiger Esterverbindungen gemäß den Ansprüchen 1 bis 4 gelöst.

Die erfindungsgemäßen Esterverbindungen haben die allgemeine Formel (**Ia**), **(Ib), (Ic)**, wie in den Ansprüchen definiert.

Im Gegensatz zu den bekannten sogenannten Estolid-Verbindungen mit der Struktur **Lit-1,** die in Abbildung 1 dargestellt ist, besitzen die erfindungsgemäßen Verbindungen mit der allgemeinen Struktur (**Ia**), **Ib**), **(Ic)** eine zusätzliche "Methylenbrücke" (Methylen-Einheit, -(CH₂)-) zwischen dem Kohlenstoff der Fettsäurekette und dem Sauerstoff der C-O-Einfachbindung der benachbarten Estergruppe. Diese "Methylenbrücke" eröffnet zusätzliche Optionen hinsichtlich konformativer Änderungen der jeweiligen Moleküle und kann somit zu einer höheren sterischen Flexibilität beitragen, verbunden mit entsprechend vorteilhaften Produkteigenschaften. Dies eröffnet auch die Möglichkeit zu neuartigen Produkteigenschaften im Vergleich zu anderen, rigideren Schmierstoffen wie beispielsweise die sogenannten Estolid-Verbindungen. Im Gegensatz zu den Estolid-Verbindungen, die laut Beschreibung durch Verknüpfung mehrerer ungesättigter Fettsäuren infolge nicht-kontrollierter Addition der Carbonsäure-Einheit einer ungesättigten Fettsäure mit der Alken-Einheit einer weiteren ungesättigten Fettsäuren hergestellt wird und entsprechend zu Gemischen von Verbindungen unterschiedlicher Kettenlänge führt, gelingt mit Hilfe des erfindungsgemäßen Verfahrens die gezielte Herstellung von Verbindungen bestimmter Kettenlänge, wobei diese als Regiosiomere vorliegen. Diese selektive Synthese von Verbindungen unterschiedlicher Kettenlänge und deren Vorliegen in isolierter Form ermöglicht das spätere gezielte *"blending",* also das für die angestrebte individuelle Anwendung maßgeschneiderte Mischen von Verbindungen unterschiedlicher Kettenlänge zum Erreichen der gewünschten Schmierstoff-Eigenschaften. Die individuellen Vertreter der erfindungsgemäßen Verbindungen bilden somit ein modulares "Baukasten-System", das gezielt für spezifische Schmierstoff-Anwendungen dann eingesetzt werden kann.

Als besonders bevorzugte Vertreter der erfindungsgemäßen Verbindungen der allgemeinen Formel (**Ia**), **(Ib), (Ic)** gelten solche Verbindungen, die ausgehend von den ungesättigten Fettsäuren Ölsäure, Linolsäure, Linolensäure, Erucasäure, Nervonsäure, Gadoleinsäure und anderen ω-*n*-Fettsäuren hergestellt werden können.

Als ein beispielhafter Verbindungstyp dieser besonders bevorzugten Vertreter der erfindungsgemäßen Verbindungen mit der allgemeinen Struktur (**I**) seien dabei die nachfolgend in Abbildung 2 dargestellten und von Ölsäure abgeleiteten Verbindungen der allgemeinen Struktur (la) genannt, wobei m' gleich 1 bis 5, n' und n" entweder 1 und 2 oder 2 und 1, R² und R⁶, die in Anspruch 1 beschriebenen Bedeutungen besitzen.

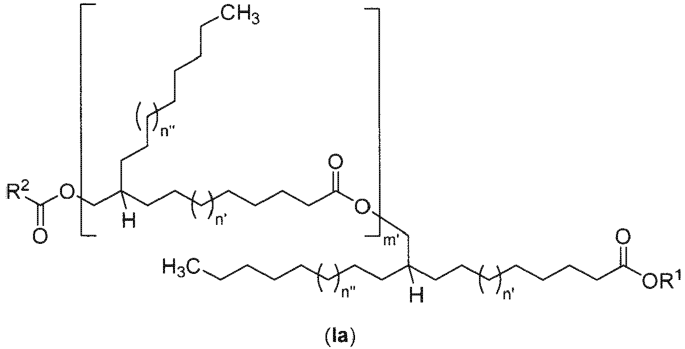

### Abbildung 2. Besonders bevorzugte Vertreter der erfindungsgemäßen Verbindungen am Beispiel der Ölsäurederivate (Ia)

Ein weiterer Verbindungstyp der besonders bevorzugten Vertreter der erfindungsgemäßen Verbindungen mit der allgemeinen Struktur (**I**) sind die nachfolgend in Abbildung 3 dargestellten und von Ölsäure abgeleiteten Verbindungen mit **"Diolbrücken"** der allgemeinen Struktur **(Ib),** wobei m' gleich 0 bis 5, n' und n" entweder 1 und 2 oder 2 und 1, n‴ gleich 0 bis 10 sind, R² und R⁶, die in Anspruch 2 beschriebenen Bedeutungen besitzen

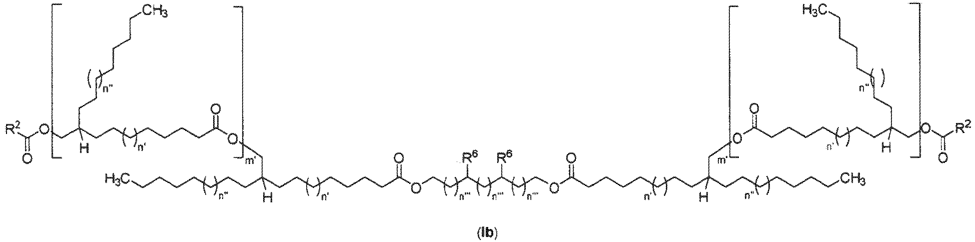

### Abbildung 3. Besonders bevorzugte Vertreter der erfindungsgemäßen Verbindungen am Beispiel der Ölsäurederivate (Ib)

Ein alternativer, weiterer beispielhafter Verbindungstyp der besonders bevorzugten Vertreter der erfindungsgemäßen Verbindungen mit der allgemeinen Struktur (**I**) seien dabei die nachfolgend in Abbildung 4 dargestellten und von Ölsäure abgeleiteten Verbindungen mit **"Dicarbonsäurebrücken"** der allgemeinen Struktur (**Ic**), wobei m' gleich 0 bis 5, n' und n" entweder 1 und 2 oder 2 und 1, n‴ gleich 0 bis 10 sind, R² und R⁶, die in Anspruch 3 beschriebenen Bedeutungen besitzen.

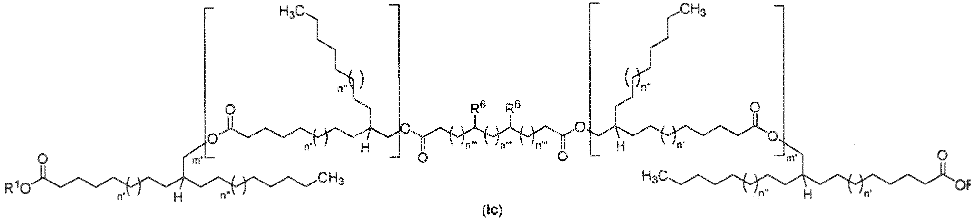

### Abbildung 4. Besonders bevorzugte Vertreter der erfindungsgemäßen Verbindungen am Beispiel der Ölsäurederivate (Ic)

Die Herstellung der neuartigen erfindungsgemäßen Verbindungen kann auf vielfältigem Wege erfolgen, wobei nachfolgend zwei Verfahren beschrieben werden, wobei eine Ausführungsform bevorzugt ist. Die Synthesen unterscheiden sich dabei erheblich von denen zu anderen Schmierstoff-Verbindungen führenden Herstellverfahren, zum Beispiel im Vergleich zu den Verfahren zur Herstellung der sogenannten Estolid-Verbindungen, auf deren Wege typischerweise die erfindungsgemäßen Verbindungen nicht zugänglich sind.

Beide Ausführungsformen der Synthese der erfindungsgemäßen Verbindungen gehen von einer ungesättigten Fettsäure als Ausgangsverbindung aus. Insbesondere kommen hierbei Ölsäure, Linolsäure, Linolensäure, Erucasäure und Nervonsäure, Gadoleinsäure und anderen ω-*n*-Fettsäuren zum Einsatz. Der Einsatz von nachwachsenden Rohstoffen als Ausgangsverbindungen ist hierbei aus der Perspektive der Ökonomie als auch Nachhaltigkeit vorteilhaft.

Für die beiden oben genannten zwei bevorzugten Synthesestrategien gibt es dabei jeweils unterschiedliche Ausführungsformen. Insbesondere kann die Sequenz der dabei beteiligten Reaktionsschritte in beliebiger Form variiert werden.

Die beiden oben genannten zwei bevorzugten Synthesestrategien werden im Nachfolgenden im Detail beschrieben.

In der ersten Ausführungsform geht das Verfahren von einer ungesättigten Fettsäure aus, die zunächst verestert wird oder es wird direkt der entsprechende Ester eingesetzt. Dieses veresterte Produkt wird dann in Gegenwart von molekularem Wasserstoff (H₂) sowie Kohlenmonoxid (CO) einer Hydroformylierungsreaktion unterzogen und nach anschließender Hydrierung, die vorzugsweise *in situ* erfolgt, wird das korrespondierende Methylol-substituierten Derivat erhalten. Diese Schritte der Hydroformylierung ungesättigter Fettsäuren bzw. deren anschließende Hydrierung zum korrespondierenden Methylol-substituierten Derivat sind vielfach in der Literatur beschrieben, beispielsweise in R. Lai, M. Naudet, E. Ucciani, Rev. Fr. Corps Gras 1966, 13, 737-745, R. Lai, M. Naudet, E. Ucciani, Rev. Fr. Corps Gras 1968, 15, 5-21, R. Lai, E. Ucciani, M. Naudet, Bull. Soc. Chim. Fr. 1969, 793-797, E. N. Frankel, J. Am. Oil Chem. Soc. 1971, 48, 248-253, E. N. Frankel, F. L. Thomas, J. Am. Oil Chem. Soc. 1972, 49, 10-14, J. P. Friedrich, G. R. List, V. E. Sohns, J. Am. Oil Chem. Soc. 1973, 50, 455-458, E. H. Pryde, J. Am. Oil Chem. Soc. 1984, 61, 419-425, E. H. Pryde, J. Am. Oil Chem. Soc. 1984, 61, 419-425 und E. Benetskiy, S. Lühr, M. Vilches-Herrera, D. Selent, H. Jiao, L. Domke, K. Dyballa, R. Franke, A. Börner, ACS Catal. 2014, 4, 2130-2136.

Als Übersichtsartikel zu diesem Thema stehen dem Fachmann zudem beispielsweise die Beiträge von E. H. Pryde, E. N. Frankel, J. C. Cowan, J. Am. Oil Chem. Soc. 1972, 49, 451-456, E. N. Frankel, Ann. N.Y. Acad. Sci. 1973, 214, 79-93, J. W. E. Coenen, Fette, Seifen, Anstrichmittel 1975, 77, 461-467, E. N. Frankel, E. H. Pryde, J. Am. Oil Chem. Soc. 1977, 54, 873A-881A, E. H. Pryde, J. Am. Oil Chem. Soc. 1979, 56, 719A-725A zur Verfügung.

Die Hydroxy-Gruppe des erhaltenen Methylol-substituierten Derivats wird anschließend erneut verestert, wobei dies beispielsweise direkt mit einer nichthydroxysubstituierten Fettsäure erfolgen kann oder mit einer anderweitigen langkettigen, mindestens eine Hydroxy-Gruppe aufweisende Alkancarbonsäure (oder deren Ester im Sinne einer Umesterungsreaktion). Diese langkettigen, mindestens eine Hydroxy-Gruppe aufweisende Alkancarbonsäure stellt dabei vorzugsweise eine langkettige Fettsäure dar, die mindestens eine Hydroxymethyl-Gruppe trägt und beispielsweise auf oben geschildertem Weg hergestellt werden kann. Für die Veresterung der Hydroxy-Gruppe zur Herstellung der Dimeren bzw. Oligomeren können entweder die jeweiligen Säuren direkt oder aktivierte Säurederivate, z.B. Säurechloride und Anhydride, oder Säureester (für eine Umesterungsreaktion) eingesetzt werden. Es können auch Di-, Tri- und höhere Carbonsäuren bzw. deren Derivate eingesetzt werden.

Im Detail umfasst die erste Ausführungsform des Verfahrens zur Herstellung von erfindungsgemäßen Esterverbindungen die folgenden Schritte:
(A) Ausgehend von einer ungesättigten Fettsäure bzw. einem daraus abgeleiteten Ester mit der allgemeinen Formel (IV) 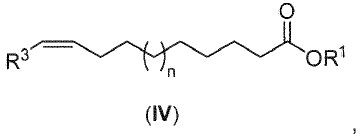
   wobei R¹, R³ und n wie oben genannt definiert sind,
   wird eine Hydroformylierung unter Ausbildung von Verbindungen mit der allgemeinen Formel (**V**) 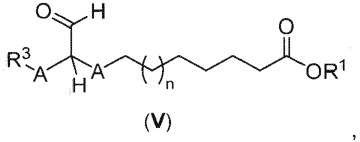
   wobei R¹, R³ und n wie oben genannt definiert sind, und A ausgewählt ist aus CH₂, CH₂-CH₂, cis-CH=CH und/oder trans-CH=CH durchgeführt, und
(B) die erhaltenen Verbindungen mit der allgemeinen Formel (**V**) werden anschließend durch Hydrierung in die Verbindungen mit der allgemeinen Formel (**VI**) 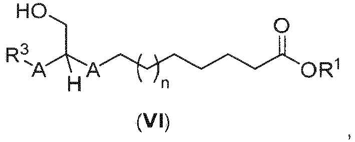 wobei R¹, R³, A und n wie oben genannt definiert sind,
   umgewandelt, und
(C) anschließend wird eine Esterbildungsreaktion unter Verwendung eines Acyldonors mit der Formel (**VII**) 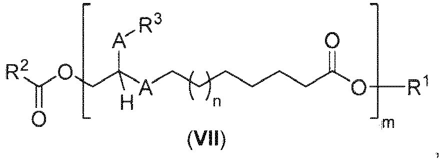 wobei R¹, R², R³, A und n sowie m wie oben genannt definiert sind, durchgeführt, wodurch die erfindungsgemäßen Verbindungen erhalten werden. Optional kann anschließend noch ein Austausch der R¹-Gruppe durch eine Veresterungs- bzw. Umesterungsreaktion erfolgen. Ebenfalls optional kann im Falle einer freien Hydroxy-Gruppe im Rest R² noch dessen weitere Veresterung erfolgen.

Diese erste bevorzugte Ausführungsform sei nachfolgend zudem am Beispiel der Herstellung entsprechender erfindungsgemäßen Verbindungen ausgehend von Ölsäure als exemplarischer Vertreter einer ungesättigten Fettsäure als Ausgangsverbindung vorgestellt bzw. verdeutlicht. Diese Syntheseroute ist zudem graphisch in nachfolgender Abbildung 5 zusammenfassend dargestellt.

Ausgehend von Ölsäure 1 als eine aus einem nachwachsenden Rohstoff einfach zugängliche Ausgangsverbindung erfolgt zunächst eine Veresterungs-reaktion, wobei im exemplarischen Beispiel 2-Ethylhexan-1-ol als gut verfügbare und in großen Mengen erhältliche Bulkchemikalie verwendet wird (Abbildung 5). Für die Veresterung steht dem Fachmann generell ein breites Spektrum an Methoden zur Verfügung, wobei eine attraktive Syntheseoption katalytische Verfahren unter Einsatz von Säuren oder Biokatalysatoren, vorzugsweise einer Lipase, darstellen.

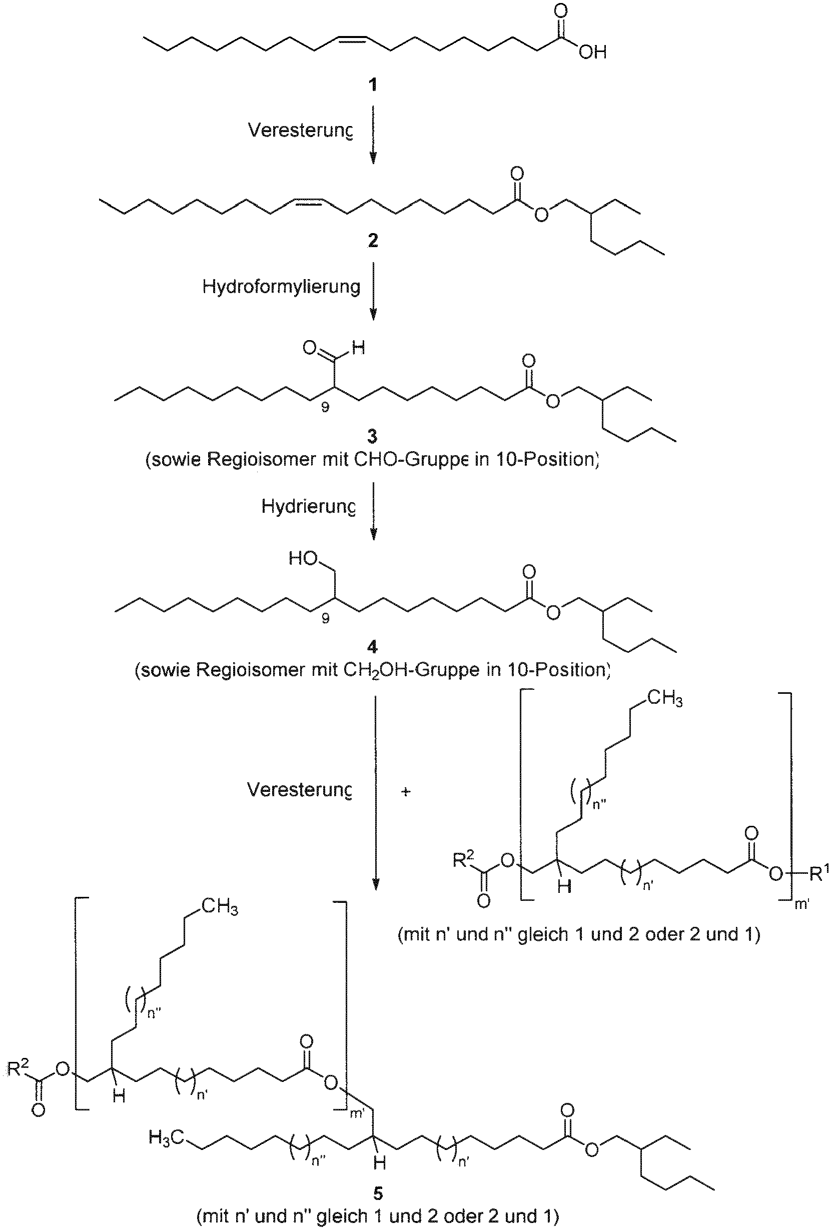

### Abbildung 5. Bevorzugte Synthesestrategien ausgehend von Ölsäure und unter Einbezug einer Hydroformylierung zur Herstellung von Verbindungen 5

Der bei der Veresterungsreaktion erhaltene Ester **2** wird anschließend einer Hydroformylierungsreaktion unterzogen, wodurch die in 9-Position mit einem CH(=O)-Rest substituierte Verbindung **3** (im Gemisch mit dem in 10-Position mit einem CH(=O)-Rest substituierten Regioisomer) entsteht. Die nachfolgende Hydrierung der Carbonylgruppe in **3** liefert dann den in 9-Position Methylol-substituierten Ölsäureester **4** (im Gemisch mit dem in 10-Position Methylol-substituierten Regioisomer), der anschließend durch Acylierung der Hydroxy-Gruppe in die gewünschten Zielverbindungen **5** umgesetzt wird.

Die Reaktionsschritte Hydroformylierung und Hydrierung können dabei kombiniert werden, so dass nach der Hydroformylierung *in situ* direkt die anschließende Hydrierung der Aldehyd-Gruppe zum Methylol-substituierten Derivat **4** erfolgt. Die bei der Hydroformylierung entstehende Hydroxymethyl-Gruppe (Methylol-Gruppe) kann dabei aufgrund der typischerweise gering bzw. nicht ausgeprägten Regioselektivität der Hydroformylierungsreaktion sich sowohl in 9- als auch in 10-Position befinden und typischerweise wird ein Gemisch aus beiden Isomeren enthalten. In Abbildung 5 ist graphisch aus Gründen der Übersichtlichkeit nur das in 9-Position substituierte Isomer gezeigt. Bei den gebildeten Produkten vom Typ **5** sind Verbindungen mit m' gleich 0 oder 1 bis 5 besonders bevorzugt.

Alternativ gelingt die Herstellung der Zielverbindungen auch auf dem Wege einer zweiten, bevorzugten Ausführungsform auf nachfolgend beschriebenem Wege. Hierbei erfolgt zunächst die Reaktion einer ungesättigten Fettsäure mit Formaldehyd (oder einem Formaldehydderivat, zum Beispiel Paraformaldehyd) im Rahmen einer En-Reaktion, optional einer nachgeschalteten Reduktion der resultierenden C=C-Bindung (zum Beispiel über eine heterogenkatalytische Hydrierung), sowie gefolgt von einer nachfolgenden Veresterung. Die Sequenz kann auch hier variiert werden und beispielsweise zuerst mit der Veresterung begonnen werden, gefolgt von der En-Reaktion und nachgeschalteter Veresterung. Für die Veresterung der Hydroxy-Gruppe zur Herstellung der Dimeren bzw. Oligomeren können entweder Carbonsäuren direkt oder aktivierte Carbonsäurederivate, z.B. Carbonsäurechloride und Anhydride, oder Carbonsäureester (für eine Umesterungsreaktion) eingesetzt werden. Es können auch Di-, Tri- und höhere Carbonsäuren bzw. deren Derivate eingesetzt werden.

Der Schritt der En-Reaktion ausgehend von einer ungesättigten Fettsäure und Formaldehyd (oder einem Formaldehydderivat, zum Beispiel Paraformaldehyd) unter Ausbildung der Hydroxymethyl-substituierten ungesättigten Fettsäurederivat-Produkte mit der *trans*-C=C-Doppelbindung in benachbarter Position zum Hydroxymethyl-Substituenten ist in der Literatur bereits beschrieben, beispielsweise in U. Biermann, J. Metzger, Fat. Sci. Technol. 1991, 93, 282-284 und J. Metzger, U. Biermann, Synthesis 1992, 5, 463-465, so dass der Fachmann sich bei der Auswahl der Reaktionsbedingungen an diesen beschriebenen Arbeiten orientieren kann. So wird beispielsweise in U. Biermann, J. Metzger, Fat. Sci. Technol. 1991, 93, 282-284 bei der Umsetzung von Ölsäure als Fettsäure mit Paraformaldehyd (2.3 Äquivalente) noch Me₂AlCl als in stöchiometrischen Mengen zugesetzte Lewis-Säure mit ebenfalls 2.3 Äquivalenten verwendet. Die En-Reaktion mit anderweitigen Substraten wurde bereits früher berichtet, unter anderem in B. Snider, D. Rodini, T. Kirk, R. Cordova, J. Am. Chem. Soc. 1982, 104, 555-563, so dass vom Fachmann bei der Wahl der Reaktionsbedingungen auch die in diesen Arbeiten beschriebenen Reaktionsbedingungen herangezogen werden können.

Im Detail umfasst diese zweite Ausführungsform des Verfahrens zur Herstellung von erfindungsgemäßen Esterverbindungen der allgemeinen Formel **(I)** die folgenden Schritte:
(A) Ausgehend von einer ungesättigten Fettsäure bzw. einem daraus abgeleiteten Ester mit der allgemeinen Formel (**IV**) 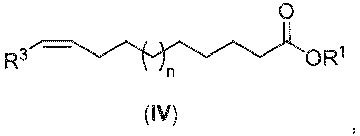
   wobei R¹, R³ und n wie oben genannt definiert sind,
   wird eine En-Reaktion und anschließender optionaler Hydrierung der dabei entstehenden C=C-Doppelbindung unter Ausbildung von Verbindungen mit der allgemeinen Formel (**VI**) 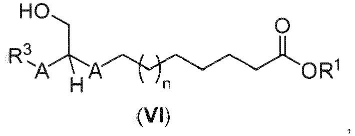
   wobei R¹, R³, A und n wie oben genannt definiert sind, durchgeführt, und
(B) die erhaltenen Verbindungen mit der allgemeinen Formel (**VI**) werden anschließend einer Esterbildungsreaktion unter Verwendung eines Acyldonors mit der Formel (**VII**) 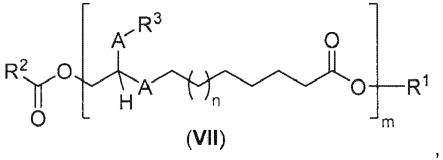 wobei R¹, R², R³, A und n sowie m wie oben genannt definiert sind, unterzogen, wodurch erfindungsgemäße Verbindungen erhalten werden. Optional kann anschließend noch ein Austausch der R¹-Gruppe durch eine Veresterungs- bzw. Umesterungsreaktion erfolgen. Ebenfalls optional kann im Falle einer freien Hydroxy-Gruppe im Rest R² noch dessen weitere Veresterung erfolgen.

Die zweite Refernzausführungsform sei im Folgenden am Beispiel der Herstellung der jeweiligen Referenzverbindungen ausgehend von Ölsäure als exemplarischer Vertreter einer ungesättigten Fettsäure als Ausgangs-verbindung vorgestellt und verdeutlicht. Dieses Beispiel ist zudem graphisch zusammenfassend in nachfolgender Abbildung 6 dargestellt. Hierbei kann die Reaktionssequenz der einzelnen beteiligten Schritte variiert werden, so dass unter anderem die im Nachfolgenden beschriebenen Referenzausführungsformen A und B daraus resultieren.

Ausgehend von Ölsäure 1 erfolgt bei der Referenzausführungsform A zunächst eine Veresterungsreaktion, wobei im exemplarischen Beispiel 2-Ethylhexan-1-ol als gut verfügbare und in großen Mengen erhältliche Bulkchemikalie verwendet wird. Wie bereits oben genannt steht für die Veresterung dem Fachmann generell ein breites Spektrum an Methoden zur Verfügung, wobei eine attraktive Syntheseoption katalytische Verfahren unter Einsatz von Säuren oder Biokatalysatoren, vorzugsweise einer Lipase, darstellen. Der bei der Veresterungsreaktion erhaltenen Ester **2** wird anschließend einer En-Reaktion mit Paraformaldehyd in Gegenwart einer Lewis-Säure unterzogen, wodurch das Hydroxymethyl-substituierte ungesättigte Fettsäurederivat **6,** enthaltend eine *trans*-C=C-Doppelbindung in benachbarter Position zum Hydroxymethyl-Substituenten (z.B. befindet sich die Doppelbindung bei Einführung der Hydroxymethyl-Funktion in 9-Position dann in 10,11-Position), entsteht (Abbildung 6). Die nachfolgende Hydrierung dieser zur Methylol-Gruppe benachbarten C=C-Doppelbindung (Alken-Einheit) in **6** liefert dann den gesättigten Methylol-substituierten Ölsäureester **4,** der anschließend durch Acylierung der Hydroxy-Gruppe in die gewünschten Zielverbindungen vom Typ **5** umgesetzt wird.

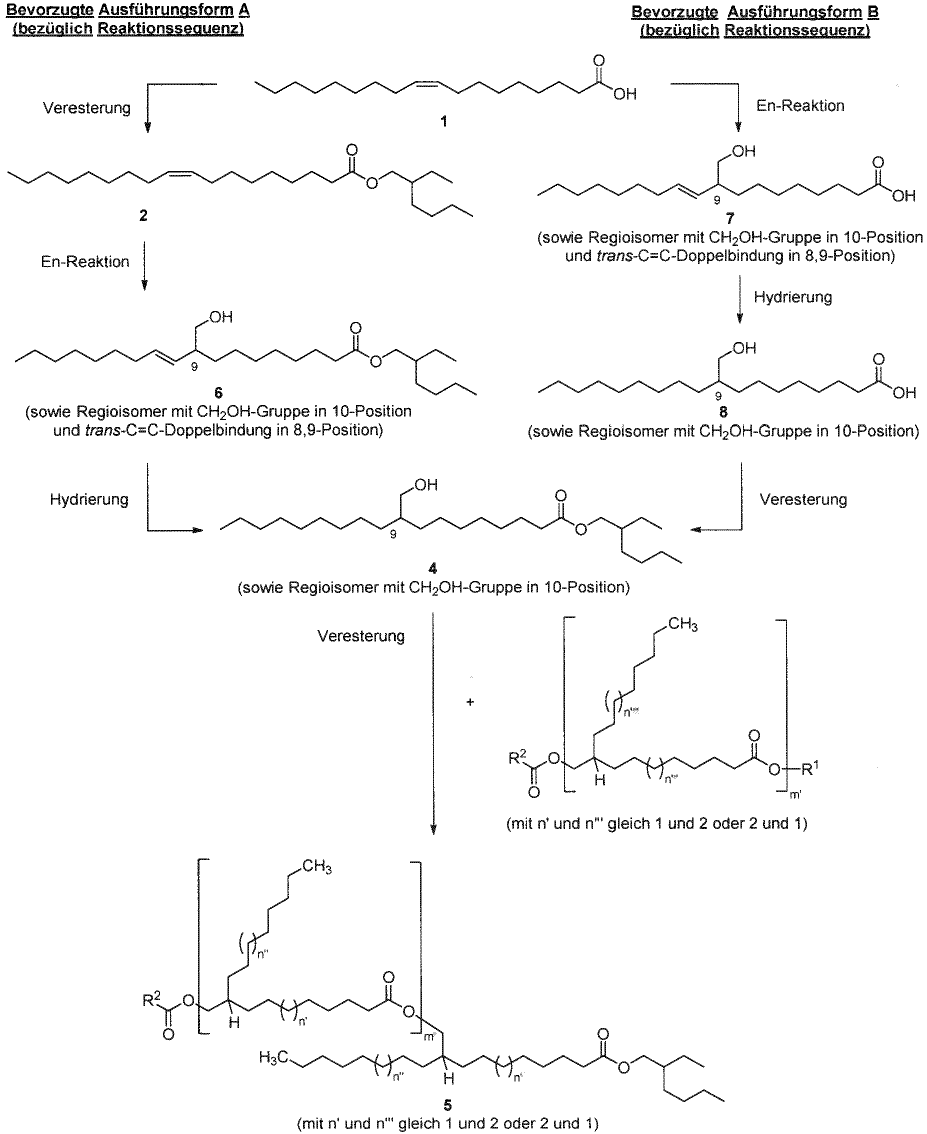

### Abbildung 6. Bevorzugte Synthesestrategien ausgehend von Ölsäure und unter Einbezug einer En-Reaktion zur Herstellung von Verbindungen 5

Die bei der En-Reaktion entstehende Hydroxymethyl-Gruppe kann dabei aufgrund der gering bzw. nicht ausgeprägten Regioselektivität der En-Reaktion sich sowohl in 9- als auch in 10-Position befinden mit entsprechender Positionierung der *trans*-C=C-Doppelbindung in 10,11-Position (im Falle der 9-Position der Hydroxymethyl-Gruppe) oder in 8,9-Position (im Falle der 10-Position der Hydroxymethyl-Gruppe). Typischerweise wird ein Gemisch aus beiden Isomeren enthalten. In Abbildung 6 ist graphisch aus Gründen der Übersichtlichkeit nur das in 9-Position Hydroxymethyl-substituierte Isomer mit *trans*-C=C-Doppelbindung in 10,11-Position gezeigt. Bei den gebildeten Produkten vom Typ **5** sind Verbindungen mit m' gleich 0 oder 1 bis 5 besonders bevorzugt.

Optional kann eine Hydrierung der aus der En-Reaktion resultierenden C=C-Doppelbindungen auch erst abschließend nach zunächst erfolgter Oligomerisierung durchgeführt werden. Darüber hinaus kann optional zudem generell auf eine oder mehrere der Hydrierungsschritte verzichtet werden, wobei die dann resultierenden Produkte mindestens eine Doppelbindung aufweisen.

Bei der ebenfalls bevorzugten Referenzusführungsform B ist im Vergleich zur Ausführungsform A die Reaktionssequenz geändert (Abbildung 6). So erfolgt zunächst direkt ausgehend von Ölsäure 1 eine En-Reaktion unter Erhalt der Verbindung **7,** welche anschließend auf dem Wege einer Hydrierung der *trans-*C=C-Doppelbindung und nachfolgender Veresterung in die Zwischenstufe **4** umgewandelt wird. Optional und alternativ kann auch zuerst eine Veresterung der Verbindung **7** (wodurch die Verbindung **6** gebildet werden würde) und schließend die Hydrierung erfolgen, wodurch ebenfalls die Verbindung **4** erhalten wird. Auch in diesem Fall der En-Reaktion ausgehend von **1** kann die entstehende Hydroxymethyl-Gruppe in Verbindung **7** aufgrund der gering bzw. nicht ausgeprägten Regioselektivität der En-Reaktion sich sowohl in 9- als auch in 10-Position befinden mit entsprechender Positionierung der *trans*-C=C-Doppelbindung in 10,11-Position (im Falle der 9-Position der Hydroxymethyl-Gruppe) oder in 8,9-Position (im Falle der 10-Position der Hydroxymethyl-Gruppe). Typischerweise wird ein Gemisch aus beiden Isomeren enthalten. In Abbildung 6 ist graphisch aus Gründen der Übersichtlichkeit nur das in 9-Position Hydroxymethyl-substituierte Isomer mit *trans*-C=C-Doppelbindung in 10,11-Position gezeigt. Die Verbindung **4** wird dann wie oben bereits bei der Ausführungsform A beschrieben in die gewünschten Zielverbindungen vom Typ **5** umgewandelt, wobei Verbindungen mit m' gleich 0 oder 1 bis 5 besonders bevorzugt sind.

Ein beispielhafter Vertreter der bevorzugten Verbindungsklasse (**Ib**) ist die nachfolgend beschriebene Mischung der drei durch Umesterung der in dem nachfolgend genannten Beispiel 9 beschriebenen Mischung von zwei Regioisomeren mit 1,6-n-Hexandiol als (Di-)Alkoholkomponente (Abbildung 7). Da 1,6-*n*-Hexandiol ein Diol darstellt und somit zweifach verestert werden kann und hierbei mit 1,6-n-Hexandiol sowohl das in 9- als auch in 10-Position substituierte Regioisomer der in Beispiel 9 hergestellten Ester-Mischung reagieren kann, entsteht hierbei eine Mischung aus 3 Regiosiomeren (durch Beteiligung von zwei 9-substituierten Regioisomeren bzw. zwei 10-substituierten Regioisomeren bzw. einem 9-substituierten Regioisomer und einem 10-substituierten Regioisomer in einer solchen Umesterungsreaktion), die in nachfolgender Abbildung 7 dargestellt ist. Zur besseren Veranschaulichung ist das jeweils aus 1,6- n-Hexandiol hervorgehende Molekülfragment umrahmt dargestellt. Alternativ kann die Herstellung solcher Vertreter der Verbindungsklasse (**Ib**) auch durch die Umesterung ausgehend von einfachen Alkylestern (wie Methyl- und Ethylestern) oder direkt ausgehend von den korrespondieren Carbonsäuren durch eine Veresterungsreaktion erfolgen.

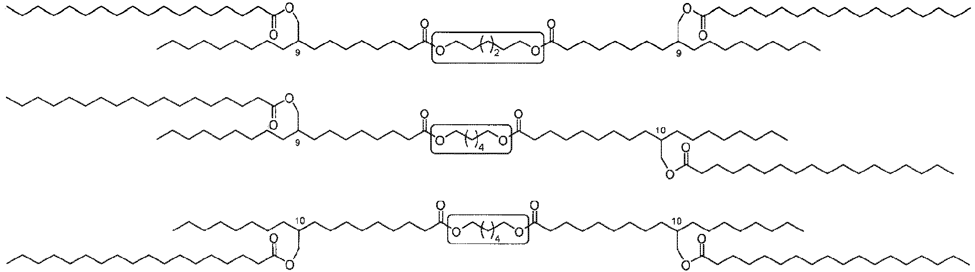

### Abbildung 7. Besonders bevorzugter Vertreter der erfindungsgemäßen Verbindungen gemäß der bevorzugten allgemeinen Struktur (Ib), hergestellt ausgehend von 1,6-Hexandiol als Diol-Komponente

Als ein beispielhafter Vertreter der bevorzugten Verbindungsklasse **(Ic)** sei die nachfolgend beschriebene Mischung der drei durch Veresterung der in nachfolgenden Beispiel 8 beschriebenen Mischung von zwei Regioisomeren mit n-Hexandicarbonsäure als (Di-)Carbonsäurekomponente genannt (Abbildung 8). Da n-Hexandicarbonsäure eine Dicarbonsäure darstellt und somit zweifach verestert werden kann und hierbei mit n-Hexandicarbonsäure sowohl das in 9-als auch in 10-Position substituierte Regioisomer der in Beispiel 8 hergestellten Ester-Mischung reagieren kann, entsteht hierbei eine Mischung dann aus 3 Regiosiomeren (durch Beteiligung von zwei 9-substituierten Regioisomeren bzw. zwei 10-substituierten Regioisomeren bzw. einem 9-substituierten Regioisomer und einem 10-substituierten Regioisomer in einer solchen Umesterungsreaktion), die in nachfolgender Abbildung 8 dargestellt ist. Zur besseren Veranschaulichung ist das jeweils aus n-Hexandicarbonsäure hervorgehende Molekülfragment umrahmt dargestellt. Alternativ kann die Herstellung solcher Vertreter der Verbindungsklasse **(Ic)** auch durch die Umesterung ausgehend von analogen *O-*acylierten Verbindungen mit strukturell einfacheren Acyl-Komponenten (wie zum Beispiel Acetyl, Propanoyl) oder direkt ausgehend von den korrespondieren nicht O-acylierten Verbindungen mit freier Hydroxymethyl-Gruppe durch eine Veresterungsreaktion erfolgen.

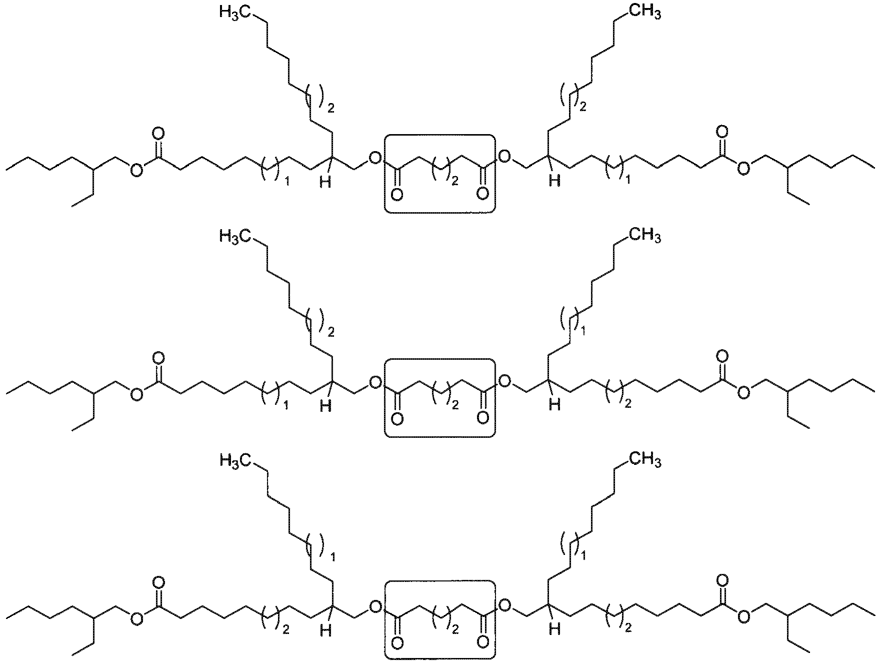

### Abbildung 8. Besonders bevorzugter Vertreter der erfindungsgemäßen Verbindungen gemäß der bevorzugten allgemeinen Struktur (Ic), hergestellt ausgehend von n-Hexandicarbonsäure als Dicarbonsäure-Komponente

Des weiteren ist ein Vertreter dieser Verbindungsklasse (**Ic**) die in dem nachfolgenden Beispiel 13 beschriebene Mischung aus den darin enthaltenen, der allgemeinen Struktur (**Ic**) entsprechenden Regiosomeren, die aus der Veresterungsreaktion der in Beispiel 8 beschriebenen erfindungsgemäßen Mischung mit der Dicarbonsäure Pripol 1013 der Firma Croda GmbH gebildet wird (Abbildung 9). Zur besseren Veranschaulichung ist das jeweils aus der kommerziellen Dicarbonsäure Pripol 1013 hervorgehende Molekülfragment umrahmt dargestellt.

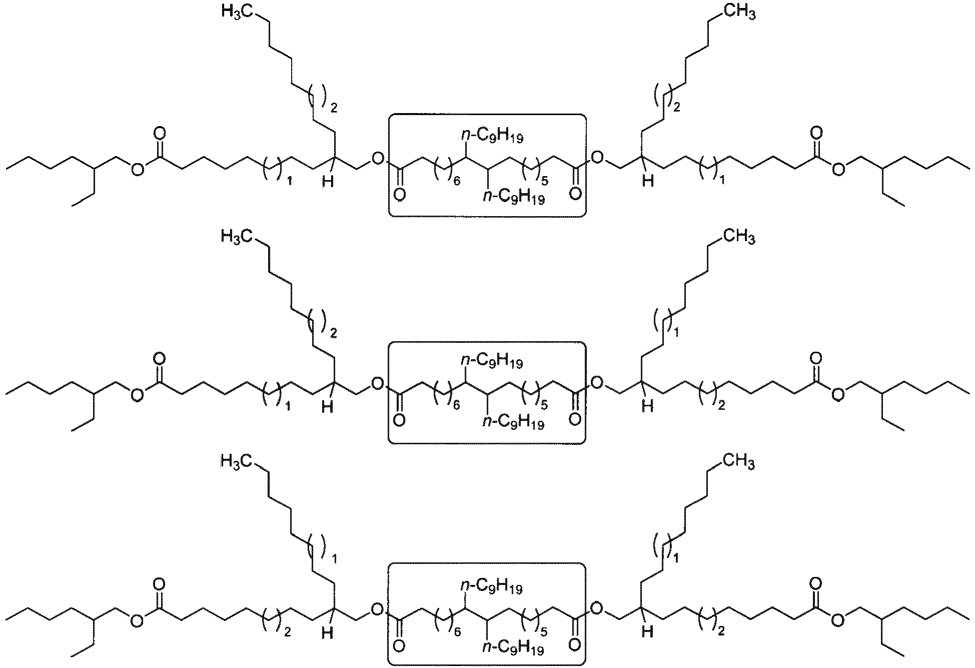

### Abbildung 9. Besonders bevorzugter Vertreter der erfindungsgemäßen Verbindungen gemäß der bevorzugten allgemeinen Struktur (Ic), hergestellt ausgehend von der Dicarbonsäure Pripol 1013 als Dicarbonsäure-Komponente

Neben *n*-Hexandicarbonsäure und Dimersäure (Pripol 1013) werden weitere mehrwertige Carbonsäuren und Carbonsäureanhydride verwendet, insbesondere hydrierte Dimersäure, hydrierte oder nicht hydrierte Trimersäuren, Terephthalsäure, Isophthalsäure, Phthalsäure, Trimellitsäure, Hemimellitsäure, Trimesinsäure, Zitronensäure, Itaconsäure, Oxalsäure, 2,2'-Thiodiessigsäure, 3,3'-Thiodipropionsäure, Admerginsäure, 2,5-Furandicarbonsäure, Cyclohexan-1,4-dicarbonsäure, Cyclohexan-1,2-dicarbonsäure, Cyclohexen-4,5-dicarbonsäure, Phenylbernsteinsäure, Glutaminsäure, Asparaginsäure, Ethylendiamintetraessigsäure, Diethylentriaminpentaessigsäure, Propylendiamintetraessigsäure, Nitrilotriessigsäure, Diglycolsäure und Iminodiessigsäure und deren Derivate.

Ein weiteres Kennzeichen des Verfahrens ist der Einbezug des Reaktionstyps der Veresterung im Rahmen der von ungesättigten Fettsäuren ausgehenden bevorzugten Ausführungsformen des Verfahrens und der dabei angewendeten Synthesesequenzen. Für die Veresterung von Hydroxy-Gruppen stehen dem Fachmann vielfältige Möglichkeiten zur Verfügung. So können entweder die benötigten Säuren direkt oder davon abgeleitete, aktivierte Säurederivate, z.B. Säurechloride und Anhydride, oder Säureester (für eine Umesterungsreaktion) eingesetzt werden. Eine besonders attraktive Syntheseoption stellen dabei katalytische Verfahren dar, die eine Direktveresterung ausgehend von Alkohol und Säure-Komponente ohne Notwendigkeit des Einsatzes von aktivierten Carbonsäurederivaten wie Carbonsäurechloride und Anhydride erlauben. Solche katalytischen Verfahren sind sowohl unter Beteiligung von Chemokatalysatoren bzw. Biokatalysatoren bekannt und beispielweise (insbesondere mit Fokus auf Biokatalysatoren) in G. Hills, Eur. J. Lipid Sci. Technol. 2003, 105, 601-607, O. Thum, K. M. Oxenboll, SOFW J. 2008, 134, 44-47, L. Hilterhaus, O. Thum, A. Liese, Org. Process Res. Dev. 2008, 12, 618-625 und M. B. Ansorge-Schumacher, O. Thum, Chem. Soc. Rev. 2013, 42, 6475-6490 beschrieben. Der Einsatz von Biokatalysatoren ist dabei besonders attraktiv, da unter milden Reaktionsbedingungen Veresterungsreaktionen in hocheffizienter Weise durchgeführt werden können. Entsprechend stellt die Herstellung der erfindungsgemäßen Verbindungen der allgemeinen Formel (**I**) im Rahmen eines Syntheseverfahrens, bei der für mindestens eine der dabei beteiligten Veresterungsreaktionen ein Biokatalysator ausgewählt aus der Enzymklasse der Hydrolasen verwendet wird, eine besonders bevorzugte Ausführungsform dar. Besonders geeignet als Biokatalysatoren aus der Enzymklasse der Hydrolasen sind für das erfindungsgemäße Verfahren dabei Lipasen, wobei wiederum die kommerziell verfügbare Lipase aus *Candida antarctica* B eine besonders geeignete Lipase-Komponente darstellt.

Für die zu den erfindungsgemäßen Verbindungen der allgemeinen Formel (**Ia**), (**Ib**)**,** (**Ic**) führenden Syntheseverfahren stehen dem Fachmann bei der Wahl der Reaktionsbedingungen vielfältige Wahlmöglichkeiten zur Verfügung, wobei für die beteiligten Einzelreaktionstypen diese in der Literatur eingehend beschrieben sind. Unter anderem ist der Fachmann frei bei der Wahl der jeweiligen Reaktionsmedien und kann beispielsweise auf ein breites Spektrum an Lösungsmitteln zur Durchführung der Synthesereaktionen des erfindungsgemäßen Verfahrens zurückgreifen. Besonders vorteilhaft ist dabei allerdings im Hinblick auf die Nachhaltigkeit des Herstellverfahrens die Vermeidung von Lösungsmitteln. Insbesondere für Veresterungsreaktionen sind Lösungsmittel-freien Synthesen in der Literatur bereits als effizient beschrieben, beispielsweise in den bereits oben genannten Beiträgen G. Hills, Eur. J. Lipid Sci. Technol. 2003, 105, 601-607, O. Thum, K. M. Oxenboll, SOFW J. 2008, 134, 44-47, L. Hilterhaus, O. Thum, A. Liese, Org. Process Res. Dev. 2008, 12, 618-625 und M. B. Ansorge-Schumacher, O. Thum, Chem. Soc. Rev. 2013, 42, 6475-6490. Da der Reaktionstyp der Veresterung auch ein Kennzeichen des erfindungsgemäßen Verfahrens darstellt, ist die Herstellung der erfindungsgemäßen Verbindungen der allgemeinen Formel (**I**) im Rahmen eines Syntheseverfahrens, bei der mindestens eine der dabei beteiligten Veresterungsreaktionen unter Lösungsmittel-freien Reaktionsbedingungen erfolgt, eine besonders bevorzugte Ausführungsform.

Ein besonderer technischer Vorteil des entwickelten Verfahrens sowie der damit erhaltenen Produkte mit einer O-acylierten Methylol-Funktion ist das Vorliegen einer primären Alkoholfunktion bei den zugrundeliegenden Hydroxymethylolsubstituierten Fettsäureestern als Substrat. Diese funktionelle primäre AlkoholGruppe ist in hohem Maße für Enzym-katalytische Reaktionen unter milden Herstellungsbedingungen und bei niedrigen Temperaturen geeignet und ermöglicht dadurch die Herstellung der gewünschten Verbindungen unter umweltfreundlichen Bedingungen bei gleichzeitigem niedrigem Energieverbrauch.

Ein weiterer genereller technischer Vorteil der erfindungsgemäßen Verbindungen sowie des Herstellungsverfahrens liegt in der enorm hohen Selektivität. Die in der DE 698 35 694 T2 offenbarten Estolidverbindungen können nicht in dieser Selektivität hergestellt werden, da hier ausgehend von einer ungesättigten Fettsäure als Basisbaustein durch Addition der Fettsäureeinheit dieses Bausteins an die Alkeneinheit des nächsten Bausteins die Reaktionsverknüpfung erfolgt und dadurch Di-, Tri-, Tetra- bzw. generell Oligomere und sogar Polymere als Mischung aufgebaut werden. Bei dem erfindungsgemäßen Verfahren wird durch milde Reaktionsbedingungen und hochselektive Enzymreaktionen, aber auch durch die Strategie der Reaktionsführung vorzugsweise jeweils nur die Mono-Funktionalisierung der Methylol-Komponente der Ausgangsverbindung erreicht (die beispielsweise als Mischung aus einem 9- und 10-Methylol-substituierten Fettsäureester besteht). Darüber hinaus ist auch die maßgeschneiderte Herstellung höher substituierte Trimere und Tetramere in selektiver Art und Weise durch entsprechend gezielte Synthesen denkbar. Daher werden die Zielverbindungen mit hoher Selektivität in definierter Form unter Vermeidung von höheren oligomeren oder polymeren Strukturen erhalten, wie dies mit den bekannten Verfahren, mit denen komplexe Produktmischungen erhalten werden, nicht möglich ist.

Die erfindungsgemäßen Esterverbindungen der allgemeinen Formel (**Ia**)**,** (**Ib**)**,** (**Ic**) sind hervorragend geeignet, in Schmierstoffzusammensetzungen eingesetzt zu werden und eignen sich sowohl zum Einsatz im Hochtemperaturbereich, im Marinebereich sowie als Schmiermittel, das im Lebensmittelbereich eingesetzt wird.

Neben den neuen Esterverbindungen können die Schmiermittelzusammensetzungen weitere Grundölkomponenten insbesondere auf der Basis von natürlichen Glyceridestern und Fettsäuren enthalten, vorzugsweise Sonnenblumenöl, Rapsöl oder Rüböl, Leinöl, Maisöl oder Maiskeimöl, Diestelöl, Sojabohnenöl, Leinsamenöl, Erdnussöl, "Lesqueralle"-Öl, Palmöl, Olivenöl, in der monomeren, oligomeren und/oder polymerisierten Formen oder Mischungen aus den genannten Ölen.

Darüber hinaus können die Schmiermittelzusammen-setzungen neben den neuen Esterverbindungen weitere Ester wie Trimethylolpropan- und Pentaerytritolester sowie TMP- Komplexester, vollständig oder teilverestert mit gesättigten und/oder einfach oder mehrfach ungesättigten Carbonsäuren der Kettenlänge C6-C36, wobei diese linear oder verzweigt sein können, Komplexester aus Dimersäuren, Dimersäureester wie Ethylhexyldimerat, aliphatische Carbonsäure- und Dicarbonsäureester sowie Phosphatester, Trimellith- und Pyromellithsäureester, Ether, Polyetherpolyole sowie Perfluorpolyether, Alkyldiphenylether und Polyphenylether, Silikonöle, Polyglycole bestehend aus statistisch verteilten Polyoxyethylen- und/oder Polyoxypropyleneinheiten und/oder andere Polyoxyalkylenbausteine sowie anderer Glycolderivate, Polyalphaolefine und Metallocen-katalysiert hergestellte Polyalphaolefine sowie Alphaolefin-Copolymere. polymere Systemen wie beispielsweise nicht hydriertes, teilhydriertes oder vollhydriertes Polyisobutylen oder einer Mischung dieser, Styrol- und Polystyrol sowie seine Derivate und/ oder polymere Systeme basierend auf Acrylaten, Acetatpolymeren und Amiden, Polyethylenen, Polypropylenen, halogenierten Polypropylenen und/ oder Cycloalkanen, Mineralöle wie z.B. Weißöl, alkylierte Diphenylether, alkylierte Naphtaline und Perfluoropolyether enthalten.

Das Schmiermittel, das die erfindungsgemäße Esterverbindung der allgemeinen Formel (**Ia**), (**Ib**)**,** (**Ic**) enthält, kann sowohl in Form eines Schmieröls als auch als Schmierfett eingesetzt werden.

Das Schmiermittel umfasst des weiteren Additive, die einzeln oder in Kombination eingesetzt werden und aus der Gruppe bestehend aus Korrosionsschutzadditiven, Antioxidaten, Verschleißschutzadditiven, UV-Stabilisatoren, anorganischen oder organischen Feststoffschmierstoffen, Pourpoint- und VI-Verbesserern, Polymeren, Haftzusätzen, Farbstoffen, Emulgatoren, Entschäumern und Festschmierstoffen ausgewählt werden, die für die Formulierung eines Schmieröles bzw. Schmierfettes typisch sind.

Schmierfette können mit unterschiedlichen Verdickungsmitteln hergestellt werden. Eine mögliche Verdickergruppe sind Harnstoffe bestehend aus dem Reaktionsprodukt aus einem Diisocyanat, vorzugsweise 2,4-Diisocyanatotoluol, 2,6-Diisocyanatotoluol, 4,4'-Diisocyanatodiphenylmethan, 2,4'-Diisocyanatophenylmethan, 4,4'-Diisocyanatodiphenyl, 4,4'-Diisocyanato-3-3'-dimethylphenyl, 4,4'-Diisocyanato-3,3'-dimethylphenylmethan, die einzeln oder in Kombination verwendet werden können, mit einem Amin der allgemeinen Formel R'₂-N-R, oder einem Diamin der allgemeinen Formel R'₂-N-R-NR'₂, wobei R ein Aryl-, Alkyl- oder Alkylenrest mit 2 bis 22 Kohlenstoffatomen ist und R' identisch oder verschieden ein Wasserstoff, ein Alkyl-, Alkylen- oder Arylrest ist, oder mit Gemischen aus Aminen und Diaminen oder als Verdickungsmittel wird ein Vertreter ausgewählt aus der Gruppe der Al-Komplexseifen, Metall-Einfachseifen der Elemente der ersten und zweiten Hauptgruppe des Periodensystems, Metall-Komplexseifen der Elemente der ersten und zweiten Hauptgruppe des Periodensystems, Bentonite, Sulfonate, Silikate, Aerosil, Polyimide oder PTFE oder einer Mischung der vorgenannten Verdickungsmittel.

Um den gesetzlichen Bestimmungen bezüglich der Verwendung von Schmierstoffen zum Schmieren von Arbeitsgeräten für die Verarbeitung von Lebensmitteln zu entsprechen, ist es zweckmäßig, wenn die eingesetzten Additive eine H1-Klassifikation aufweisen.

Der Zusatz von Antioxidantien kann die Oxidation des Öls oder Fetts, insbesondere bei seinem Einsatz, verringern oder gar verhindern. Entsprechend stellt der Zusatz von Antioxidantien eine weitere bevorzugte Ausführungsform des erfindungsgemäßen Verfahrens dar. Die Antioxidantien werden ausgewählt aus der Gruppe bestehend aus diaromatischen Aminen, Phenolharzen, Thiophenolharzen, Phosphiten, butyliertem Hydroxytoluol, butylierten Hydroxyanisol, Phenyl-α-naphthylaminen, Phenyl-β-naphthylaminen, octylierten/butylierten Diphenylaminen, Di-α-Tocopherol, Di-tert-butyl-phenyl, Benzolpropansäure und Mischungen dieser Komponenten.

Der Schmierstoff kann Korrosionsschutzadditive, Metalldesaktivatoren oder lonen-Komplexbildner enthalten. Hierzu zählen Triazole, Imidazoline, N-Methylglycin (Sarcosin), Benzotriazolderivate, *N,N*-Bis(2-ethylhexyl)-ar-methyl-1*H*-benzotriazol-1-methanamin; *N*-Methyl-*N*-(1-oxo-9-octadecenyl)glycin, Gemisch aus Phosphorsäure und deren Mono- und Diisooctylester umgesetzt mit (C11-14)-Alkylaminen, Gemische aus Phosphorsäure und Mono- und Diisooctylester umgesetzt mit *tert*-Alkylaminen und primären (C12-14)-Aminen, Dodekansäure, Triphenylphosphorthionat und Aminphosphate. Kommerziell erhältliche Additive sind beispielsweise die nachfolgend genannten Produkte: IRGAMET^{®} 39, IRGACOR^{®} DSS G, Amin O; SARKOSYL^{®} O (Ciba), COBRATEC^{®} 122, CUVAN^{®} 303, VANLUBE^{®} 9123, CI-426, CI-426EP, CI-429 und CI-498.

Der Schmierstoff kann zudem Verschleißschutzadditive, Anti-Wear- Additive und Frictionmodifier enthalten.

Verschleißschutzadditive sind Amine, Aminphosphate, Phosphate, Thiophosphate, Phosphorthionate, Arylphosphat, alkylierte Polysulfide, geschwefelte Aminverbindungen, geschwefelte Fettsäuremethylester, Naphthensäuren, Nanopartikel aus den Gruppen Al₂O₃, SiO₂, TiO₂, ZrO₂, WO₃, Ta₂O₅, V₂O₅, CeO₂, Aluminiumtitanat, BN, MoSi₂, SiC, Si₃N₄, TiC, TiN, ZrB₂, Tonminerale und/oder deren Gemische sowie thermisch stabile Carbonate und / oder Sulfate, und Mischungen dieser Komponenten. Zu den kommerziell erhältlichen Verschleißschutzadditiven gehören IRGALUBE^{®} TPPT, IRGALUBE^{®} 232, IRGALUBE^{®} 349, IRGALUBE^{®} 211 und ADDITIN^{®} RC3760 Liq 3960, FIRC-SHUN^{®} FG 1505 und FG 1506, NA-LUBE^{®} KR-015FG, LUBEBOND^{®}, FLUORO^{®} FG, SYNALOX^{®} 40-D, ACHESON^{®} FGA 1820 und ACHESON^{®} FGA 1810.

Der Schmierstoff kann Pourpoint- und Viskositätsverbesser und Haftzusätze enthalten. Pourpoint- und Viskositätsverbesser sind ausgewählt aus den Gruppen der linearen und/oder verzweigten alkylierten, acrylierten und aliphatischen Polymeren und Copolymeren sowie polymerisierten Fettsäureestern, so wie aus der Gruppe PIB (Polyisobutylene) und PB (Polybutene) teilhydriert oder vollhydriert.

Der Schmierstoff kann desweiteren UV-Stabilisatoren enthalten. UV-Stabilisatoren sind ausgewählt aus den Gruppen der Stickstoffheterocyclen, substituierten Stickstoffheterocyclen, linear und verzweigten alkylierten, acylierten, aliphatischen Stickstoffheterocyclen, sowie derer Derivate.

Der Schmierstoff kann auch Festschmierstoffe enthalten. Festschmierstoffe sind z.B. PTFE, BN, Pyrophosphat, Zn-Oxid, Mg-Oxid, Pyrophosphate, Thiosulfate, Mg-Carbonat, Ca-Carbonat, Ca-Stearat, Zn-Sulfid, Mo-sulfid, W-sulfid, Sn-Sulfid, Graphite, Graphen, Nano-Tubes, SiO2-Modifikationen oder eine Mischung daraus enthalten.

Der Schmierstoff kann Emulgatoren enthalten. Emulgatoren sind ausgewählt aus den Gruppen der verzweigten und/oder linearen ethoxylierten und/oder propoxylierten Alkohole und deren Salze wie beispielsweise Alkohole, C16-C18, ethoxyliert, propoxyliert, Polyglykole, Fettsäureester, Silikate, ionische Tenside wie z. B. Natriumsalze von Alkylsulfonsäuren, wobei die Ketten C14-17-Kohlenstoffe enthalten.

Der Schmierstoff kann Entschäumer enthalten. Entschäumer sind ausgewählt aus den Gruppen der ethoxylierten und/oder propoxylierten Alkohole der Kettenlängen C10-C18, Mono- und Diglyceride von Speisefetten, Akrylate, propoxylierte und/oder ethoxylierte Alkylether (Polyglycole), Alkohole, Siloxane.

Eine bevorzugte Form des Herstellungsverfahren für eine Ölformulierung ist dabei wie folgt: Die Esterverbindung wird im Kessel vorgelegt. Die viskositätsgebende Komponente und/oder ein oder mehrere weitere Grundöle werden zugegeben, und unter Rühren und gegebenenfalls Aufheizen auf eine definierte Temperatur, eine klare Lösung erzeugt. Feste Additive werden sodann bei einer Temperatur oberhalb ihres Schmelzpunktes zugegeben und solange gerührt bis diese gelöst sind. Anschließend wird der Kesselinhalt auf maximal 60°C abgekühlt und die flüssigen Additive zugegeben. Nach einer weiteren Stunde Rührzeit kann das Öl abgefüllt werden.

Bei einer bevorzugten Form des Herstellungsverfahren für eine Fettformulierung wird zudem wie folgt vorgegangen: Die Grundölmischung wird im Kessel vorgelegt. Die Verdickerkomponenten werden unter Rühren bei einer definierten Temperatur auf definierte Art und Weise zugegeben. Das so entstehende Fett wird eine definierte Zeit gerührt und speziell bei Verwendung von Verdickungsmitteln auf Seifenbasis wasserfrei gekocht. Feste Additive werden sodann bei einer Temperatur oberhalb ihres Schmelzpunktes zugegeben und solange gerührt bis diese gelöst sind. Anschließend wird der Kesselinhalt auf maximal 60°C abgekühlt und die flüssigen Additive zugegeben. Nach einer weiteren Stunde Rührzeit kann das Fett abgefüllt werden.

Die Schmiermittelzusammensetzungen auf Basis der Esterverbindung der allgemeinen Formeln (**Ia**) oder (**Ib**) oder (**Ic**), werden im Marinebereich, im Bereich der Binnengewässer und bei Offshore-Anlagen, d.h. zur Schmierung von Ketten, Gleitlagern, Propellerrudern, Propellerwellen, Maschinenbauteilen und Anlagen, die im Marinebereich mit Salzwasser oder in Binnengewässern mit Wasser und wässrigen Medien in Berührung kommen, eingesetzt. Des weiteren finden sie Anwendung bei der Schmierung von Arbeitsgeräten in der lebensmittelverarbeitenden Industrie, als Hydrauliköl in der lebensmittelverarbeitenden Industrie, für Transport- und Steuerketten, für Vorrichtungen für die Verarbeitung von Getreide, Mehl und Tierfutter, sowie in Backöfen. Außerdem werden sie zur Schmierung von Wälz- und Gleitlagern, Transport- und Steuerketten in der Fahrzeugtechnik, der Fördertechnik, dem Maschinenbau, der Bürotechnik sowie in industriellen Anlagen und Maschinen, in den Bereichen der Haushaltsmaschinen und der Unterhaltungselektronik eingesetzt. Darüber hinaus werden sie zur Schmierung von Kegelrad- und Stirnradgetrieben von Laufrollenlagern in Stranggießanlagen und Transportrollenlagern in Durchlauföfen und zur offenen Zahnkrankschmierung an Drehrohröfen, Rohrmühlen, Trommeln und Mischern, wie sie speziell in der Zement-, Kalk-, Gips-, Minen- und Chemieindustrie, eingesetzt.

Ergänzend sei angemerkt, dass es sich bei den erfindungsgemäßen Verbindungen, sofern diese Stereozentren enthalten, sowohl um racemische als auch enantiomerenangereicherte bzw. enantiomerenreine Verbindungen handeln kann.

Im nachfolgenden Abschnitt werden die erfindungsgemäßen Esterverbindungen und ihre Herstellung sowie ihre Verwendung in einer Schmierstoffzusammensetzung anhand entsprechender experimenteller Beispiele erläutert.

### Beispiele:

Allgemeine Versuchsvorschrift 1 (AVV1): Biokatalytische Synthese von Ölsäureestern ausgehend von Ölsäure und Guerbet-Alkoholen

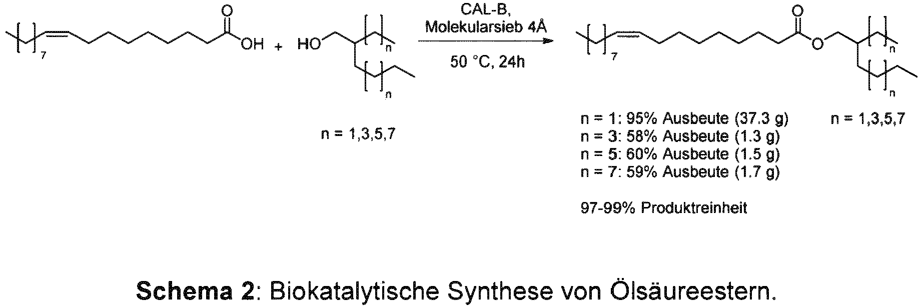

Ölsäure (1,0 Äq.) und Guerbet-Alkohol (1,0 Äq.) wurden vorgelegt und mit CAL-B (Novozym 435, 30 mg/mmol Substrat) und Molekularsieb 4Å (120 mg/mmol) versetzt. Die Reaktionsmischung wurde für 24 Stunden bei 50 °C gerührt und anschließend durch einen 0,2 µM PTFE-Filter filtriert. Der entsprechende Ölsäureester wurde in 97-99% Produktreinheit erhalten.

### Beispiel 1

### Herstellung von 2-Ethylhexyloleat ausgehend von Ölsäure und 2-Ethylhexan-1-ol

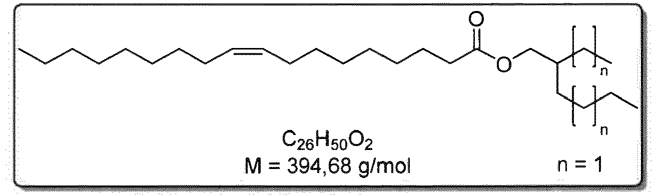

Die Synthese erfolgte nach AW 1. Ölsäure (31,6 ml, 100 mmol) und 2-Ethylhexan-1-ol (15,6 ml, 100 mmol) wurden mit Novozym 435 (3,0 g) und Molekularsieb 4Ä (12,0 g) versetzt. 2-Ethylhexyloleat (99% Reinheit) wurde als farblose Flüssigkeit erhalten.

**Ausbeute:** 37,3 g, 95%.

**¹H-NMR** (500 MHz, CDCl₃): δ [ppm] = 5.34 (m, 2H, *CH=CH),* 3.98 (dd, 2H, ²*J* = 5.8 Hz, ³*J* = 2.4 Hz, OC*H*₂), 2.29 (t, 2H, ³*J* = 7.5 Hz, CH₂C*H*₂COOR), 2.01 (m, 4H, C*H*₂CH=CHC*H*₂), 1.61 (qi, 2H, ³*J* = 7.3 Hz, C*H*₂CH₂COOR), 1.56 (sept, 1H, ³*J* = 6.0 Hz, OCH₂C*H*), 1.28 (m, 28H), 0.88 (m, 9H, C*H*₃).

**¹³C-NMR** (125 MHz, CDCl₃): δ [ppm] = 174.21, 130.11, 129.87, 66.76, 38.90, 34.58, 32.05, 30.57, 29.91, 29.84, 29.67, 29.47, 29.33, 29.29, 29.26, 29.07, 27.36, 27.31, 25.19, 23.95, 23.12, 22.83, 14.25, 14.18.

**GC** (FID): Phenomenex ZB-5MSi, 0.5 ml/min (H2), Inj. Temp.: 300 °C, Det. Temp.: 350 °C; 300 °C -> 350 °C (5 °C/min), 350 °C für 5 min, Rt = 3,51 min.

**HRMS** (ESI): berechnet für C₂₆H₅₀O₂Na[M+Na]⁺: 417.3703, gefunden: 417.3699.

IR (neat) [cm⁻¹]: 2956, 2922, 2853, 1736, 1461, 1240, 1171, 724.

### Beispiel 2

### Herstellung von 2-Butyloctyloleat ausgehend von Ölsäure und 2-Butyloctan-1-ol

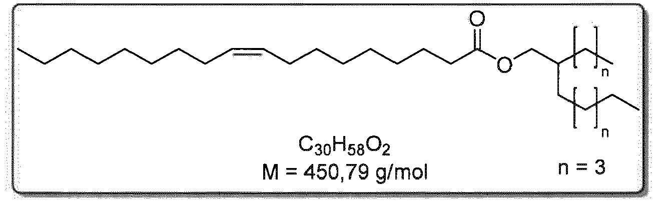

Die Synthese erfolgte nach AW 1. Ölsäure (1,59 ml, 5,00 mmol) und 2-Butyloctan-1-ol (1,12 ml, 5,00 mmol) wurden mit Novozym 435 (150 mg) und Molekularsieb 4Å (600 mg) versetzt. 2-Butyloctyloleat (97% Reinheit) wurde als farblose Flüssigkeit erhalten.

**Ausbeute:** 1,30 g, 58%.

**¹H-NMR** (500 MHz, CDCl₃): δ [ppm] = 5.34 (m, 2H, *CH*=*CH*)*,* 3.96 (d, 2H, ²*J* = 5.8 Hz, OC*H*₂), 2.29 (t, 2H, ³*J* = 7.5 Hz, CH₂C*H*₂COOR), 2.01 (m, 4H, C*H*₂CH=CHC*H*₂), 1.61 (m, 2H, C*H*₂CH₂COOR), 1.60 (m, 1H, OCH₂C*H*), 1.28 (m, 36H), 0.88 (m, 9H, C*H*₃).

**¹³C-NMR** (125 MHz, CDCl₃): δ [ppm] = 174.21, 130.11, 129.87, 67.16, 37.43, 34.60, 32.06, 31.97, 31.44, 31.11, 29.92, 29.86, 29.78, 29.68, 29.47, 29.35, 29.31, 29.28, 29.07, 27.36, 27.32, 26.82, 25.21, 23.14, 22.83, 22.81, 14.25, 14.24, 14.19.

**GC (FID):** Phenomenex ZB-5MSi, 0.5 ml/min (H2), Inj. Temp.: 300 °C, Det. Temp.: 350 °C; 300 °C -> 350 °C (5 °C/min), 350 °C für 5 min; Rt = 4,27 min.

**HRMS** (ESI): berechnet für C₃₀H₅₈O₂Na [M+Na]⁺: 473.4329, gefunden: 473,4324.

IR (neat) [cm⁻¹]: 2954, 2922, 2853, 1737, 1457, 1241, 1169, 723.

### Beispiel 3

### Herstellung von 2-Hexyldecyloleat ausgehend von Ölsäure und 2-Hexyldecan-1-ol

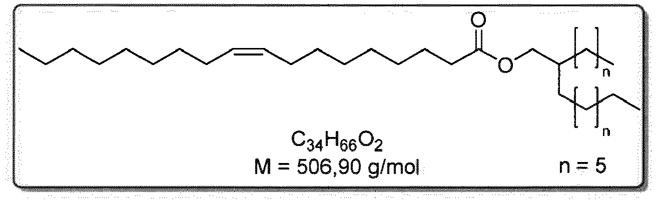

Die Synthese erfolgte nach AW 1. Ölsäure (1,59 ml, 5,00 mmol) und 2-Hexyldecan-1-ol (1,44 ml, 5,00 mmol) wurden mit Novozym 435 (150 mg) und Molekularsieb 4Å (600 mg) versetzt. 2-Hexyldecyloleat (97% Reinheit) wurde als farblose Flüssigkeit erhalten.

**Ausbeute**: 1,51 g, 60%.

**¹H-NMR** (500 MHz, CDCl₃): δ [ppm] = 5.34 (m, 2H, C*H*=C*H*), 3.97 (d, 2H, ²*J* = 5.8 Hz, OC*H*₂), 2.29 (t, 2H, ³*J* = 7.5 Hz, CH₂C*H*₂COOR), 2.01 (m, 4H, C*H*₂CH=CHC*H*₂), 1.61 (m, 2H, C*H*₂CH₂COOR), 1.60 (m, 1H, OCH₂C*H*), 1.28 (m, 44H), 0.88 (m, 9H, C*H*₃).

**¹³C-NMR** (125 MHz, CDCl₃): δ [ppm] = 174.22, 130.12, 129.87, 67.18, 37.45, 34.61, 32.06, 31.97, 31.44, 30.12, 29.92, 29.86, 29.78, 29.72, 29.68, 29.48, 29.36, 29.32, 29.29, 27.37, 27.32, 26.86, 26.82, 25.21, 22.84, 22.81, 14.26, 14.25.

**GC (FID):** Phenomenex ZB-5MSi, 0.5 ml/min (H2), Inj. Temp.: 300 °C, Det. Temp.: 350 °C; 300 °C -> 350 °C (5 °C/min), 350 °C für 5 min; Rt = 5,65 min.

**HRMS** (ESI): berechnet für C₃₄H₆₆O₂Na [M+Na]⁺: 529.4955, gefunden: 529.4951.

**IR** (neat) [cm⁻¹]: 2921, 2852, 1737, 1464, 1169, 722.

### Beispiel 4

### Herstellung von 2-Octyldodecyloleat ausgehend von Ölsäure und 2-Octyldocecan-1-ol

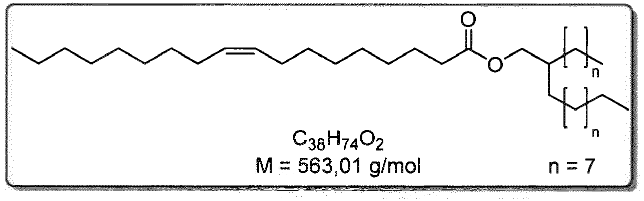

Die Synthese erfolgte nach AW 1. Ölsäure (1,59 ml, 5,00 mmol) und 2-Octyldodecan-1-ol (1,78 ml, 5,00 mmol) wurden mit Novozym 435 (150 mg) und Molekularsieb 4Å (600 mg) versetzt. 2-Octyldodecyloleat (98% Reinheit) wurde als farblose Flüssigkeit erhalten.

**Ausbeute:** 1,67 g, 59%.

**¹H-NMR** (500 MHz, CDCl₃): δ [ppm] = 5.34 (m, 2H, CH=CH), 3.97 (d, 2H, ²*J* = 5.8 Hz, OC*H*₂), 2.29 (t, 2H, ³*J* = 7.5 Hz, CH₂C*H*₂COOR), 2.01 (m, 4H, C*H*₂CH=CHC*H*₂), 1.61 (m, 2H, C*H*₂CH₂COOR), 1.60 (m, 1H, OCH₂C*H*), 1.28 (m, 52H), 0.88 (m, 9H, C*H*₃).

**¹³C-NMR** (125 MHz, CDCl₃): δ [ppm] = 174.22, 130.12, 129.87, 67.19, 37.43, 34.61, 32.08, 32.07, 31.42, 30.12, 29.92, 29.87, 29.82, 29.81, 29.77, 29.72, 29.69, 29.52, 29.48, 29.36, 29.32, 29.29, 27.37, 27.32, 26.85, 25.21, 22.84, 14.27.

**GC (FID):** Phenomenex ZB-5MSi, 0.5 ml/min (H2), Inj. Temp.: 300 °C, Det. Temp.: 350 °C; 300 °C -> 350 °C (5 °C/min), 350 °C für 5 min; Rt = 7,70 min.

**HRMS** (ESI): berechnet für C₃₄H₆₆O₂Na [M+Na]⁺: 585,5581, gefunden: 585,5568.

**IR** (neat) [cm⁻¹]: 2920, 2852, 1737, 1464, 1170, 722.

### Allgemeine Versuchsvorschrift 2 (AW2): En-Reaktion von Ölsäure/-ester mit Paraformaldehyd und Lewis-Säuren

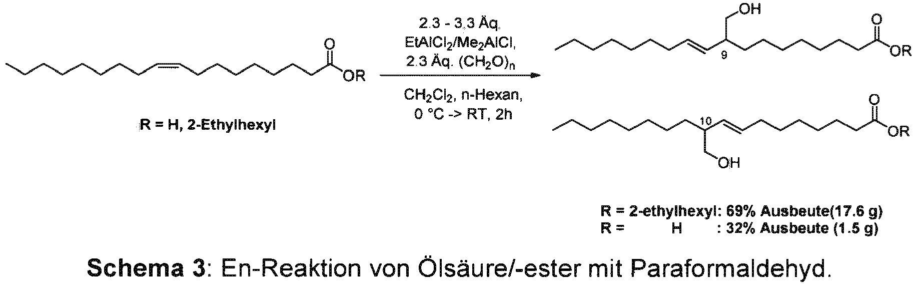

In Anlehnung an Versuchsvorschriften von Metzger und Biermann (U. Biermann, J. Metzger, Fat. Sci. Technol. 1991, 93, 282-284; J. Metzger, U. Biermann, Synthesis 1992, 5, 463-465) wurde Ölsäure (1,0 Äq.) oder sein 2-Ethylhexylester (1,0 Äq.) und Paraformaldehyd (2,3 Äq.) unter Argon in trockenem Dichlormethan vorgelegt und auf 0 °C gekühlt. Anschließend wurde EtAlCl₂ oder Me₂AlCl (2,3 - 3,3 Äq., 1,0 M in *n*-Hexan) hinzugetropft und die Reaktionsmischung danach langsam auf Raumtemperatur erwärmt und für zwei Stunden gerührt. Wasser (1:1 v/v) wurde hinzugegeben und mit 4 M HCl auf pH=1 angesäuert. Die Phasen wurden getrennt und die wässrige Phase noch dreimal mit Diethylether (1:1 v/v) extrahiert. Die vereinigten Extrakte wurden über Magnesiumsulfat getrocknet und vom Lösungsmittel bei verringertem Druck befreit. Anschließende Säulenchromatographie lieferte die Produkte als farblose Öle. Die Produkte wurden als 1:1-Gemisch der C9 und C10-Addukte erhalten.

### Beispiel 5

### Herstellung von einem Gemisch aus E-9-(Hydroxymethyl)octadec-10-ensäure und E-10-(Hydroxymethyl)octadec-8-ensäure

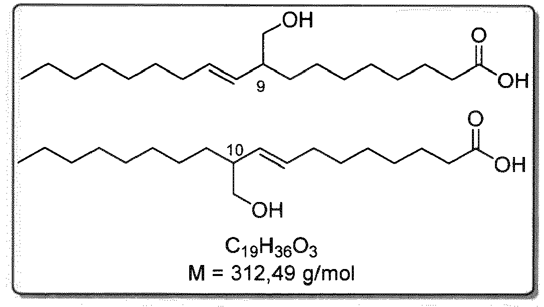

Die Synthese erfolgte nach AW 2. Ölsäure (4,73 ml, 15 mmol) und Paraformaldehyd (1,04 g, 34,5 mmol) wurden unter Zugabe von Me₂AlCl (34,5 ml, 34,5 mmol) umgesetzt. Aufarbeitung und Säulenchromatographie (Cyclohexan/Ethylacetat 7:3, v/v) lieferte das Produkt als farblose Flüssigkeit.

**Ausbeute:** 1,50 g, 32%.

**¹H-NMR** (500 MHz, CDCl₃): δ [ppm] = 5.52 (m, 1H, CH=CHCH), 5.13 (m, 1H, CH=CHCH), 3.52 (m, 1H, C*H₂*OH), 3.33 (m, 1H, C*H₂*OH), 2.34 (2 t, 2H, ³*J* = 7.5 Hz, C*H₂*COO), 2.15 (m, 1H, CH=CHCH), 2.04 (m, 2H, C*H₂*CH=CH), 1.64 (m, 2H, C*H₂*CH₂COO), 1.27 (m, 22H), 0.88 (2 t, 3H, C*H*₃).

**¹³C-NMR** (125 MHz, CDCl₃): δ [ppm] = 179.15, 179.07, 134.33, 133.88, 131.64, 131.30, 66.15, 66.10, 46.06, 34.01, 34.01, 32.82, 32.68, 32.03, 32.01, 31.27, 31.21, 29.82, 29.69, 29.68, 29.57, 29.45, 29.37, 29.29, 29.28, 29.26, 29.16, 28.99, 28.79, 27.24, 27.15, 27.07, 24.80, 24.75, 22.82, 22.81, 14.25.

Die analytischen Daten stimmen mit der Literatur (J. Metzger, U. Biermann, Synthesis 1992, 5, 463-465) überein.

### Beispiel 6

Herstellung von einem Gemisch aus E-9-(Hydroxymethyl)octadec-10-ensäure-(2'-ethylhexyl)ester und *E*-10-(Hydroxymethyl)octadec-8-ensäure-(2'-ethylhexyl)ester

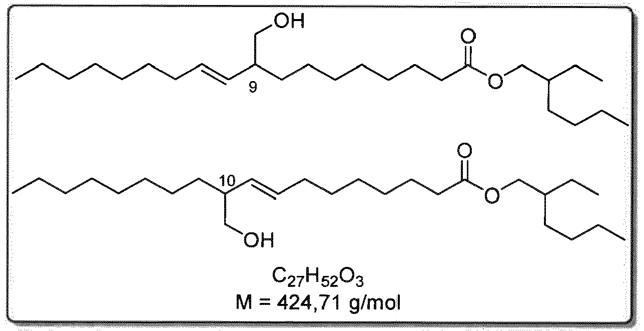

Die Synthese erfolgte nach AVV 2. 2-Ethylhexyloleat (23,7 g 60 mmol) und Paraformaldehyd (4,14 g, 138 mmol) wurden unter Zugabe von EtAlCl₂ (198 ml, 198 mmol) umgesetzt. Aufarbeitung und Vakuumdestillation (bei 10⁻³ mbar) lieferte das Produkt als farblose Flüssigkeit.

**Ausbeute:** 17,6 g, 69%.

**¹H-NMR** (500 MHz, CDCl₃): δ [ppm] = 5.51 (m, 1H, CH=CHCH), 5.12 (m, 1H, CH=CHCH), 3.98 (m, 2H, COOC*H₂*), 3.51 (m, 1H, C*H₂*OH) 3.32 (m, 1H, C*H₂*OH), 2.29 (2 t, 2H, ³*J* = 7.5 Hz, C*H₂*COO), 2.12 (m, 1H, CH=CHC*H*), 2.02 (m, 2H, C*H₂*CH=CH), 1.61 (m, 2H, C*H₂*CH₂COO), 1.56 (m, 1H, OCH₂C*H*), 1.27 (m, 28H), 0.88 (3 t, 9H, C*H*₃).

**¹³C-NMR** (125 MHz, CDCl₃): δ [ppm] = 174.21, 174.18, 134.27, 133.90, 131.58, 131.33, 66.79, 66.77, 66.12, 66.09, 46.09, 38.88, 34.56, 34.54, 32.81, 32.72, 32.02, 31.99, 31.25, 31.23, 30.56, 29.81, 29.67, 29.67, 29.64, 29.46, 29.44, 29.34, 29.28, 29.24, 29.10, 29.06, 28.90, 27.23, 27.18, 25.16, 25.12, 23.93, 23.12, 22.81, 22.80, 14.25, 14.24, 14.19, 11.13.

**GC (FID):** Phenomenex ZB-5MSi, 0.5 ml/min (H2), Inj. Temp.: 300 °C, Det. Temp.: 350 °C; 300 °C -> 350 °C (5 °C/min), 350 °C für 5 min; Rt = 4,29, 4,53 min.

**HRMS** (ESI): berechnet für C₂₇H₅₂O₃Na [M+Na]⁺: 447.3809, gefunden: 447.3813.

**IR** (neat) [cm⁻¹]: 2923, 2854, 1733, 1462, 1379, 1171, 1032, 969.

### Allgemeine Versuchsvorschrift 3 (AW3): Palladiumkatalysierte C=C-Hydrierung der ungesättigten Ölsäurederivate

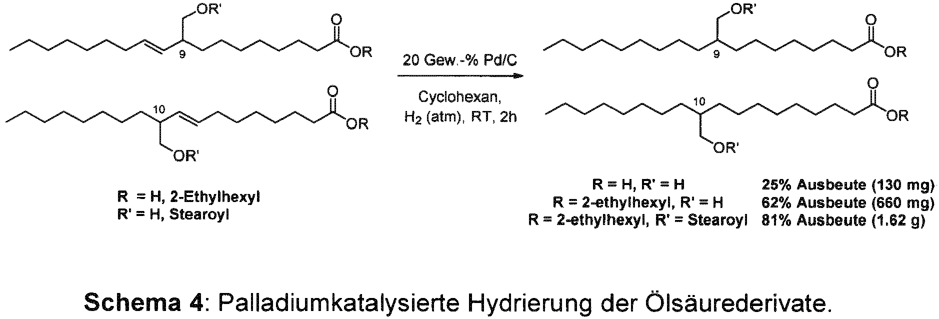

Die ungesättigte freie Säure (1,0 Äq.) oder der 2-Ethylhexylester (1,0 Äq.) wurde unter einer Wasserstoffatmosphäre in Cyclohexan gelöst und mit Palladium auf Aktivkohle (Pd/C, 10% Pd, 20 Gew.-%) versetzt. Die Reaktionsmischung wurde für zwei Stunden bei Raumtemperatur gerührt und anschließend durch einen 0,2 µM PTFE-Filter filtriert. Säulenchromatographie lieferte das gewünschte Produkt als farbloses Öl.

### Beispiel 7

### Herstellung von einem Gemisch aus 9-(Hydroxymethyl)octadecansäure und 10-(Hydroxymethyl)octadecansäure

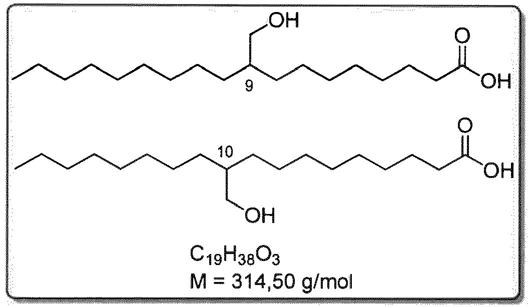

Die Synthese wurde nach AW 3 durchgeführt. Ein Gemisch aus E-9-(Hydroxymethyl)octadec-10-ensäure und *E*-10-(Hydroxymethyl)octadec-8-ensäure (450 mg, 1,44 mmol) wurde unter Wasserstoffatmosphäre in 25 ml Cyclohexan gelöst und mit Pd/C (90 mg) versetzt. Aufarbeitung und Säulenchromatographie (Cyclohexan/Ethylacetat 1:2, v/v) lieferte das gewünschte Produkt als farbloses Öl.

**Ausbeute:** 130 mg, 25%.

**¹H-NMR** (500 MHz, CDCl₃): δ [ppm] = 3.53 (d, 2H, ³*J* = 5.5 Hz C*H₂*OH), 2.35 (t, 2H, ³*J* = 7.5 Hz, C*H₂*COO), 1.63 (qi, 2H, ³*J* = 7.3 Hz, C*H₂*CH₂COO), 1.45 (m, 1H, HOCH₂C*H*), 1.27 (m, 36H), 0.88 (t, 3H, ³*J* = 6.9 Hz, C*H*₃).

**¹³C-NMR** (125 MHz, CDCl₃): δ [ppm] = 179.62, 65.81, 65.79, 40.59, 34.15, 34.14, 32.05, 31.07, 31.02, 30.99, 30.22, 30.05, 29.91, 29.80, 29.78, 29.76, 29.49, 29.30, 29.15, 29.13, 27.04, 26.92, 26.88, 24.80, 22.83, 14.27.

**HRMS** (ESI): berechnet für C₁₉H₃₈O₃Na [M+Na]⁺: 337.2713, gefunden: 337.2717.

**IR** (neat) [cm⁻¹]: 2913, 2848, 1699, 1469, 1185, 972, 719.

### Beispiel 8

### Herstellung von einem Gemisch aus 9-(Hydroxymethyl)octadecansäure-(2'-ethylhexyl)ester und 10-(Hydroxymethyl)octadecansäure-(2'-ethylhexyl)ester

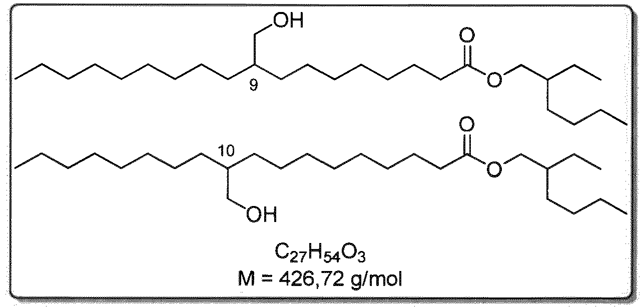

Die Synthese wurde nach AW 3 durchgeführt. Ein Gemisch aus *E*-9-(Hydroxymethyl)octadec-10-ensäure-(2'-ethylhexyl)ester und *E*-10-(Hydroxymethyl)octadec-8-ensäure-(2'-ethylhexyl)ester (1,06 g, 2,50 mmol) wurde unter Wasserstoffatmosphäre in 50 ml Cyclohexan gelöst und mit Pd/C (212 mg) versetzt. Aufarbeitung und Säulenchromatographie (Cyclohexan/Ethylacetat 7:1, v/v) lieferte das gewünschte Produkt als farbloses Öl.

**Ausbeute:** 660 mg, 62%.

**¹H-NMR** (500 MHz, CDCl₃): δ [ppm] = 3.97 (m, 2H, COOC*H₂*), 3.54 (d, 2H, ³*J* = 5.5 Hz C*H₂*OH), 2.29 (t, 2H, ³*J* = 7.5 Hz, C*H₂*COO), 1.61 (m, 2H, C*H₂*CH₂COO), 1.56 (m, 1H, OCH₂C*H*), 1.27 (m, 36H), 0.89 (3 t, 9H, C*H*₃).

**¹³C-NMR** (125 MHz, CDCl₃): δ [ppm] = 174.27, 174.26, 66.79, 65.85, 65.83, 40.67, 38.90, 34.60, 34.58, 32.05, 31.08, 31.07, 31.05, 30.57, 30.22, 30.14, 30.02, 29.81, 29.78, 29.76, 29.59, 29.49, 29.41, 29.40, 29.30, 29.07, 27.05, 27.01, 26.97, 25.19, 23.95, 23.13, 22.83, 14.27, 14.20, 11.15.

**GC (FID):** Phenomenex ZB-5MSi, 0.5 ml/min (H2), Inj. Temp.: 300 °C, Det. Temp.: 350 °C; 300 °C -> 350 °C (5 °C/min), 350 °C für 5 min; Rt = 4,61 min.

**HRMS** (ESI): berechnet für C₂₇H₅₄O₃Na [M+Na]⁺: 449.3965, gefunden: 449.3975..

**IR** (neat) [cm⁻¹]: 2921, 2853, 1736, 1459, 1171, 1031.

### Beispiel 9

### Herstellung von einem Gemisch aus 9-((Stearoyloxy)methyl)octadecansäure-(2'-ethylhexyl)ester und 10-((Stearoyloxy)methyl)octadecansäure-(2'-ethylhexyl)ester

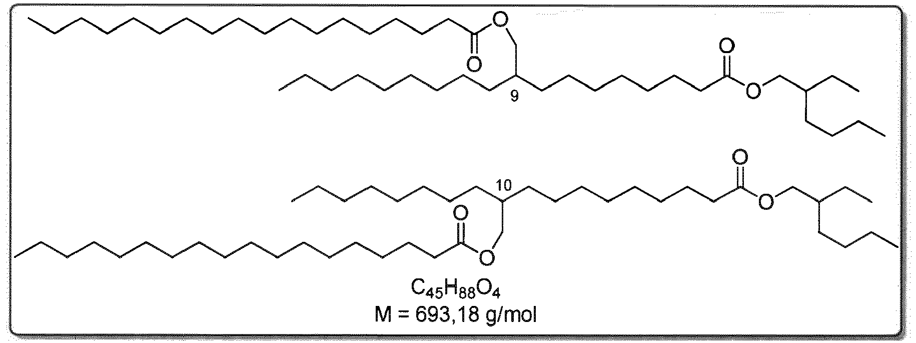

Die Synthese wurde nach AW 3 durchgeführt. Ein Gemisch aus *E*-9-(Hydroxymethyl)octadec-10-ensäure-(2'-ethylhexyl)ester und *E*-10-(Hydroxymethyl)octadec-8-ensäure-(2'-ethylhexyl)ester (2,00 g, 2,90 mmol) wurde unter Wasserstoffatmosphäre in 50 ml Cyclohexan gelöst und mit Pd/C (400 mg) versetzt. Aufarbeitung und Säulenchromatographie (Cyclohexan/Ethylacetat 15:1, v/v) lieferte das gewünschte Produkt als farbloses Öl.

**Ausbeute:** 1.62 g, 81%.

**¹H-NMR** (500 MHz, CDCl₃): δ [ppm] = 3.97 (m, 4H, COOC*H₂*), 2.28 (t, 4H, ³*J* = 7.5 Hz, C*H₂*COO), 1.61 (m, 6H, C*H₂*CH₂COO), 1.25 (m, 62H), 0.89 (m, 12H, C*H*₃).

**¹³C-NMR** (125 MHz, CDCl₃): δ [ppm] = 174.22, 174.16, 67.13, 66.75, 38.91, 37.46, 34.60, 34.58, 32.07, 31.42, 30.58, 30.12, 30.06, 29.94, 29.85, 29.81, 29.76, 29.71, 29.65, 29.63, 29.58, 29.46, 29.43, 29.39, 29.34, 29.32, 29.07, 26.85, 25.21, 23.95, 23.12, 22.84, 14.25, 14.18, 11.13.

**GC (FID):** Phenomenex ZB-5MSi, 0.5 ml/min (H2), Inj. Temp.: 300 °C, Det. Temp.: 350 °C; 300 °C -> 350 °C (5 °C/min), 350 °C für 5 min; Rt = 14,4 min.

**HRMS** (ESI): berechnet für C₄₅H₈₈O₄Na [M+Na]⁺: 715.6575, gefunden: 715.6573.

**IR** (neat) [cm⁻¹]: 2921, 2852, 1736, 1463, 1169.

**Tabelle 1**

| Chemische und physikalische Eigenschaften des Produkts hergestellt gemäß Beispiel 9 | | | |
|---|---|---|---|
| Parameter | Methode | Einheit | Produkt hergestellt gemäß Beispiel 9 |
| | | | |
| Aussehen | | | flüssig klar farblos |
| Kin. Vis. 40°C | ASTM | mm²/s | 29,8 |
| Kin. Vis. 100°C | D 7042 | | 6,56 |
| VI | | | 184,1 |
| biologische Abbaubarkeit | OECD 301 F | % | 81,3 |

### Allgemeine Versuchsvorschrift 4 (AVV4): Biokatalytische Veresterung von Fettsäuren mit hydroxymethylierten Stearinsäurederivaten zu "Dimeren"

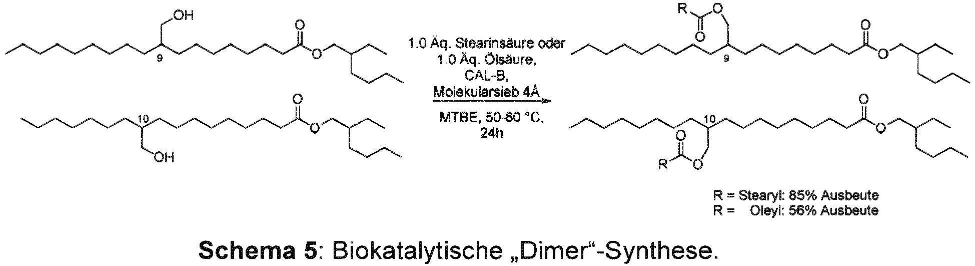

Der hydroxymethylierte Stearinsäure-(2'-ethylhexyl)ester (1,0 Äq.) wurde in MTBE gelöst und mit Novozym 435 (CAL-B, 30 mg/mmol) und Molekularsieb 4Å (120 mg/mmol) sowie einer Fettsäure (1,0 Äq.) versetzt. Die Reaktionsmischung wurde bei 50 bis 60 °C für 24 Stunden gerührt und anschließend durch einen 0,2 µM PTFE-Filter filtriert. Entfernen des Lösungsmittels unter verminderten Druck lieferte das Produkt als farbloses Öl.

### Beispiel 10

### Herstellung von einem Gemisch aus 9-((Stearoyloxy)methyl)octadecansäure-(2'-ethylhexyl)ester und 10-((Stearoyloxy)methyl)octadecansäure-(2'-ethylhexyl)ester

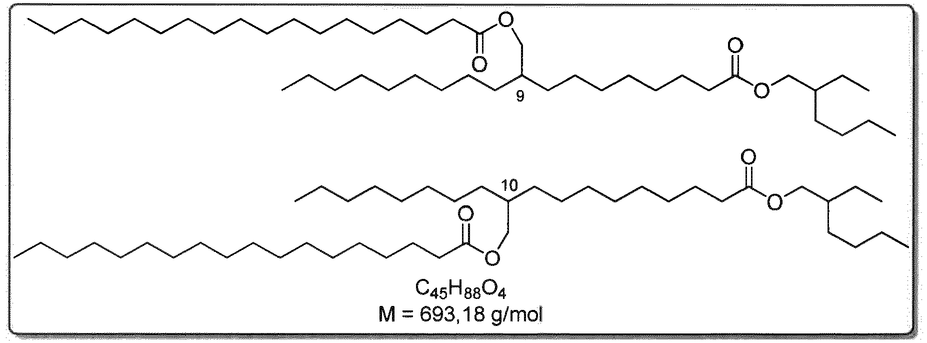

Die Synthese erfolgte nach AW 4. Ein Gemisch aus 9-(Hydroxymethyl)octadecansäure-(2'-ethylhexyl)ester und 10-(Hydroxymethyl)octadecansäure-(2'-ethylhexyl)ester (51,7 mg, 100 µmol) und Stearinsäure (28,4 mg, 100 µmol) wurden in 50 µL MTBE gelöst und mit Novozym 435 (3 mg) und Molekularsieb 4Å (12 mg) versetzt und bei 50 °C gerührt. Aufarbeitung lieferte das gewünschte Produkt als farbloses Öl.

**Ausbeute:** 58 mg, 85%.

**¹H-NMR** (500 MHz, CDCl₃): δ [ppm] = 3.98 (m, 2H, COOC*H₂*), 3.95 (m, 2H, COOC*H₂*), 2.29 (2 t, 4H, ³*J* = 7.4 Hz, C*H₂*COO), 1.61 (m, 4H, C*H₂*CH₂COO), 1.56 (m, 2H, OCH₂C*H*), 1.27 (m, 62H), 0.89 (4 t, 12H, C*H*₃).

**¹³C-NMR** (125 MHz, CDCl₃): δ [ppm] = 174.29, 174.27, 174.22, 67.16, 66.78, 38.91, 37.46, 34.63, 34.60, 34.59, 32.08, 32.07, 31.43, 30.58, 30.13, 29.86, 29.83, 29.82, 29.77, 29.72, 29.66, 29.64, 29.52, 29.51, 29.48, 29.44, 29.35, 29.33, 29.08, 26.86, 25.22, 25.21, 23.96, 23.14, 22.85, 14.28, 14.21, 11.15.

Die analytischen Daten stimmen mit denen der Verbindung aus Beispiel 9 überein.

### Beispiel 11

### Herstellung von einem Gemisch aus 9-((Stearoyloxy)methyl)octadec-10-ensäure-(2'-ethylhexyl)ester und 10-((Stearoyloxy)methyl)octadec-8-ensäure-(2'-ethylhexyl)ester

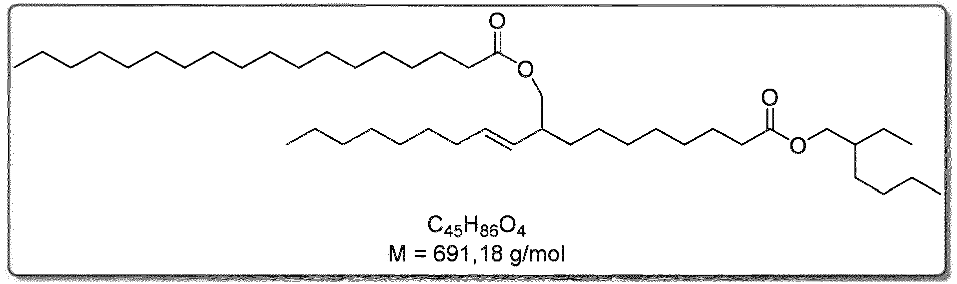

### Ein Gemisch aus E-9-(Hydroxymethyl)octadec-10-ensäure-(2'-ethylhexyl)ester und E-10-(Hydroxymethyl)octadec-8-ensäure-(2'-ethylhexyl)ester

(10,0 g, 23,5 mmol) wurde mit Stearinsäure (6,70 g, 23,5 mmol) vermischt und auf 70 °C erhitzt, wodurch die Stearinsäure schmolz. Novozym 435 (CAL-B, 706 mg, 30 mg/mmol) und Molekularsieb 4Ä (3,3 g, 120 mg/mmol) wurden hinzugefügt. Die Reaktionsmischung wurde für 24 Stunden bei 70 °C gerührt. Anschließend wurde durch ein 0.2 µM PTFE-Filter filtriert. Entfernen des Solvens *in vacuo* und Filtration über Kieselgel (Cyclohexan/Ethylacetat 15:1, v/v) lieferte das Produkt als farbloses Öl.

**Ausbeute:** 11,9 g, 73%.

**¹H-NMR** (500 MHz, CDCl₃): δ [ppm] = 5.40 (m, 1H, CH=C*H*), 5.14 (m, 1H, C*H*=CH), 3.98 (m, 2H, COOC*H₂*), 2.29 (m, 4H, C*H₂*COO), 1.97 (m, 2H, C*H*CH₂) 1.61 (m, 5H, C*H*₂CH₂COO + OCH₂C*H*), 1.27 (m, 60H), 0.88 (4 t, 12H, C*H*₃).

**GC (FID):** Phenomenex ZB-5MSi, 0.5 ml/min (H2), Inj. Temp.: 300 °C, Det. Temp.: 350 °C; 300 °C -> 350 °C (5 °C/min), 350 °C für 5 min; Rt = 14,1 min.

**HRMS** (ESI): berechnet für C₄₅H₈₆O₄Na [M+Na]⁺: 713,6418, gefunden: 713,6419.

**IR** (neat) [cm⁻¹]: 2959, 2926, 2856, 1736, 1257, 1011, 865, 790, 700.

### Beispiel 12

### Herstellung von einem Gemisch aus 9-((Octadec-9-enoyloxy)methyl)octadecansäure-(2'-ethylhexyl)ester und 10-((Octadec-9-enoyloxy)methyl)octadecansäure-(2'-ethylhexyl)ester

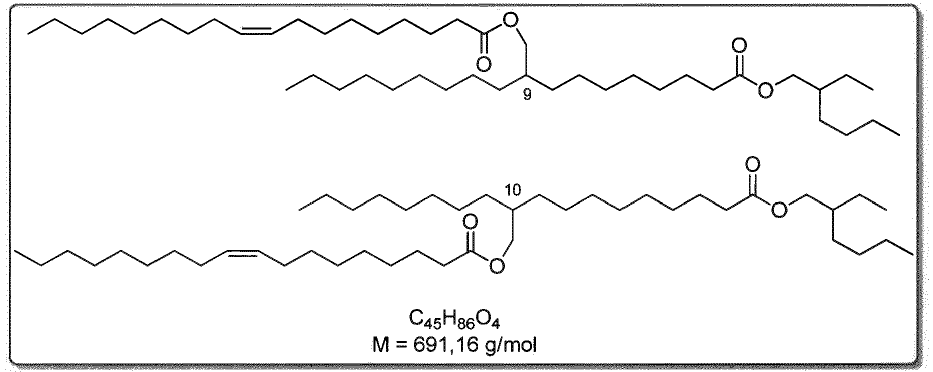

Die Synthese erfolgte nach AW 4. Ein Gemisch aus 9-(Hydroxymethyl)octadecansäure-(2'-ethylhexyl)ester und 10-(Hydroxymethyl)octadecansäure-(2'-ethylhexyl)ester (51,7 mg, 100 µmol) und Ölsäure (28,2 mg, 100 µmol) wurden mit Novozym 435 (3 mg) und Molekularsieb 4Å (12 mg) versetzt und bei 60 °C gerührt. Aufarbeitung lieferte das gewünschte Produkt als farbloses Öl.

**Ausbeute:** 39 mg, 56%.

**¹H-NMR** (500 MHz, CDCl₃): δ [ppm] = 5.34 (m, 2H, C*H*=C*H*), 3.98 (m, 2H, COOC*H₂*), 3.95 (m, 2H, COOC*H₂*), 2.29 (2 t, 4H, ³*J* = 7.4 Hz, C*H₂*COO), 1.61 (m, 4H, C*H₂*CH₂COO), 1.56 (m, 2H, OCH₂C*H*), 1.27 (m, 58H), 0.89 (4 t, 12H, C*H*₃).

**MS (ESI):** m/z = 691.5 [M+H]⁺.

**IR** (neat) [cm⁻¹]: 2959, 2926, 2856, 1736, 1257, 1011, 865, 790, 700.

### Beispiel 13

### Herstellung von einem Gemisch aus 9-(((12-Hydroxyoctadecanoyl)-oxy)methyl)octadecansäure-(2'-ethylhexyl)ester und 10-(((12-Hydroxyoctadecanoyl)-oxy)methyl)octadecansäure-(2'-ethylhexyl)ester

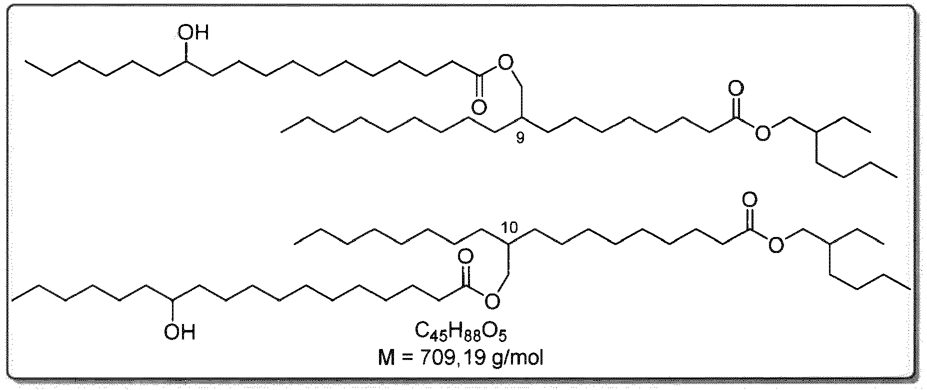

Die Synthese erfolgte nach AVV 4. Ein Gemisch aus 9-(Hydroxymethyl)octadecansäure-(2'-ethylhexyl)ester und 10-(Hydroxymethyl)octadecansäure-(2'-ethylhexyl)ester (213,2 mg; 500 µmol), und 12-Hydroxystearinsäure (142,8 mg; 480 µmol) wurden mit Molekularsieb 4 Å (59,53 mg), Novozym 435 (14,5 mg) und MTBE (0,5 mL) wurden 24 h bei 50 °C gerührt. Aufarbeitung lieferte das gewünschte Produkt als farbloses Öl.

**Ausbeute:** 267 mg, 76 %.

**¹H-NMR** (500 MHz, CDCl₃): δ [ppm] = 4.05-3,86 (m, 4H, COOC*H*₂); 3.58 (dt, 1H, ³*J* = 7.4, 4.2 Hz, C*H*₂OH); 2.29 (t, 4H, ³*J* = 7.5 Hz, C*H*₂COO); 1.58 (dt, 6H, ³*J* = 24,3, 6,6 Hz); 1.41 (d, 6H, ³*J* = 6.5 Hz); 1,37-1,20 (m, 55H); 0.88 (td, 12H, ³*J* = 6.9, 6.3, 3.8 Hz, C*H*₃).

**¹³C-NMR** (125 MHz, CDCl₃): δ [ppm] = 174.22, 150.97, 67.14, 66.78, 38.91, 37.63, 37.46, 34.59, 32.05, 31.99, 31.43, 30.58, 30.11, 29.85, 29.83, 29.75, 29.73, 29.69, 29.66, 29.61, 29.58, 29.52, 29.49, 29.46, 29.43, 29.40, 29.32, 29.30, 29.07, 26.84, 25.79, 25.76, 25.19, 23.95, 23.12, 22.82, 22.76, 14.24, 14.22, 14.18, 11.13.

**HRMS** (ESI): berechnet für C₄₅H₈₈O₅Na⁺: 731,6524; gefunden: 731,6521.

### Beispiel 14

### Herstellung von einem Gemisch aus (E)-11-((2-ethylhexyl)oxy)-2-octyl-11-oxoundec-3-en-1-yl-((E)-2-(8-((2-ethylhexyl)oxy)-8-oxooctyl)undec-3-en-1-yl)-adipinsäureester, Bis((E)-11-((2-ethylhexyl)oxy)-2-octyl-11-oxoundec-3-en-1-yl)-adipinsäureester und bis((E)-2-(8-((2-ethylhexyl)oxy)-8-oxooctyl)undec-3-en-1 - yl)-adipinsäureester

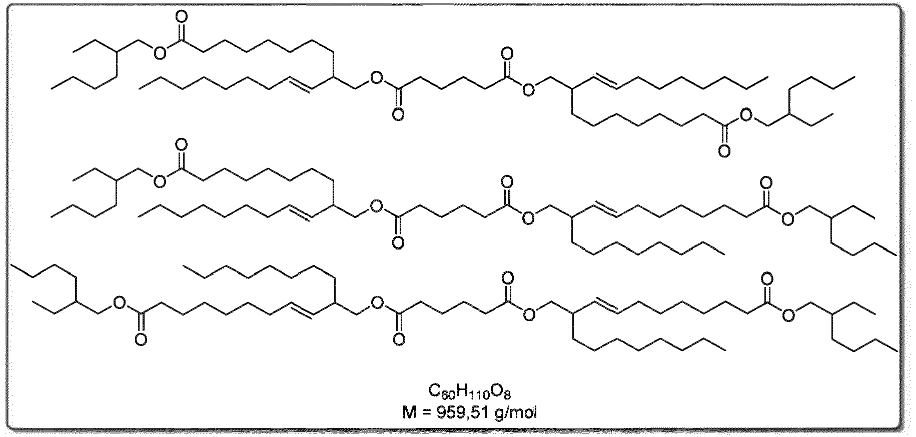

Die Synthese erfolgte in Anlehnung an AVV 4. Ein Gemisch aus E-9-(Hydroxymethyl)octadec-10-ensäure-(2'-ethylhexyl)ester und *E*-10-(Hydroxymethyl)octadec-8-ensäure-(2'-ethylhexyl)ester (999,6 mg, 2,4 mmol) und Adipinsäure (172,6 mg, 1,2 mmol) wurden mit Novozym 435 (70,6 mg) und Molekularsieb 4Ä (288 mg) versetzt und bei 60 °C gerührt. Aufarbeitung lieferte das gewünschte Produkt als gelbliches Öl.

**Ausbeute:** 1,06 g, 94%.

**¹H-NMR** (500 MHz, CDCl₃): δ [ppm] = 5.43 (m, 2H, CH=C*H*), 5.19-5.09 (m, 2H, C*H*=CH), 4.07-3.86 (m, 8H, COOC*H₂*), 2.29 (m, 8H, C*H₂*COO), 1.98 (m, 4H, C*H*CH₂) 1.61 (m, 10H, C*H*₂CH₂COO + OCH₂C*H*), 1.27 (m, 60H), 0.88 (4 t, 16H, C*H*₃)

**¹³C-NMR** (125 MHz, CDCl₃): δ [ppm] = 174.13, 174.06, 174.02, 173.53, 173.51, 173.32, 132.68, 132.62, 132.38, 132.32, 130.56, 130.44, 130.38, 67.73, 67.59, 66.80, 66.64, 42.10, 42.05, 38.76, 38.74, 34.47, 34.43, 34.39, 34.01, 33.96, 32.61, 32.57, 32.55, 31.93, 31.89, 31.46, 30.43, 30.41, 29.70, 29.65, 29.54, 29.50, 29.37, 29.32, 29.31, 29.25, 29.18, 29.17, 29.06, 29.02, 28.93, 28.80, 28.76, 26.88, 25.07, 25.04, 25.01, 24.48, 24.45, 23.81, 23.79, 22.99, 22.97, 22.70, 22.68, 14.12, 14.06, 11.00, 10.99.

**HRMS** (ESI): berechnet für C₆₀H₁₁₀O₈Na⁺: 981,8093; gefunden: 981,8094.

### Beispiel 15

### Herstellung von (Z)-Hexan-1,6-diyldioleat

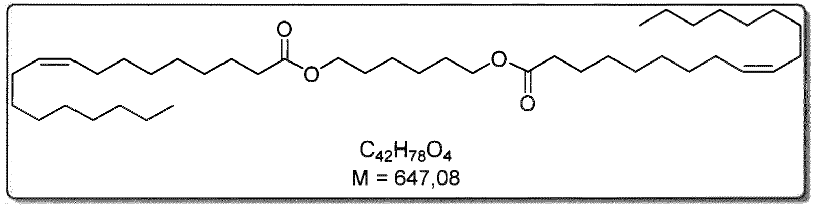

Die Synthese erfolgte in Anlehnung an Versuchsvorschriften von Raghunanan *et al.* (L. Raghunanan, S. Narine ACS Sust. Chem. & Eng. 2016 4 (3), 693-700) und Neises *et al.* (B. Neises, W. Steglich, Angew. Chem. Int. Ed. Engl. 1978, 17, 522-524). Ölsäure (5,01 g, 17,7 mmol), 1,6-Hexandiol (804,6 mg, 6,8 mmol), *N*,*N*-Dimethylaminopyridin (86,7 mg, 0,71 mmol) und *N,N'-*Dicyclohexylcarbodiimid (2,84 g, 13,8 mmol) wurden in Dichlormethan bei Raumtemperatur gerührt. Nach 18 h wurden die Phasen getrennt und die wässrige Phase noch dreimal mit Dichlormethan (1:1 v/v) extrahiert. Die vereinigten Extrakte wurden über Magnesiumsulfat getrocknet und vom Lösungsmittel bei verringertem Druck befreit. Das Produkt wurde als gelbliches Öl erhalten.

**Ausbeute:** 2,79 g, 63%.

**¹H-NMR** (500 MHz, CDCl₃): δ [ppm] = 5.34 (4H, m, C*H*=C*H*), 4.06 (4H, t, O-C*H*₂), 2.28 (4H, t, C*H₂*COO), 2.00 (8H, m, CH=CH-C*H*₂), 1.63 (8H, m, O-CH₂-C*H*₂, C*H*₂-CH₂-COO), 1.21-1.35 (44H, m), 0.88 (6H, t, CH₃).

**MS (ESI):** m/z = 669,6 [M+Na]⁺.

Die analytischen Daten stimmen mit der Literatur (L. Raghunanan, S. Narine ACS Sust. Chem. & Eng. 2016 4 (3), 693-700) überein.

### Beispiel 16

### Herstellung von einem Gemisch aus 6-(((E)-9-(Hydroxymethyl)octadec-10-enoyl)oxy)hexyl-(E)-10-(hydroxymethyl)octadec-8-ensäureester, Hexane-1,6-diyl (10E,10'E)-bis(9-(hydroxymethyl)octadec-10-ensäureester) und Hexane-1,6-diyl (8E,8'E)-bis(10-(hydroxymethyl)octadec-8-ensäureester)

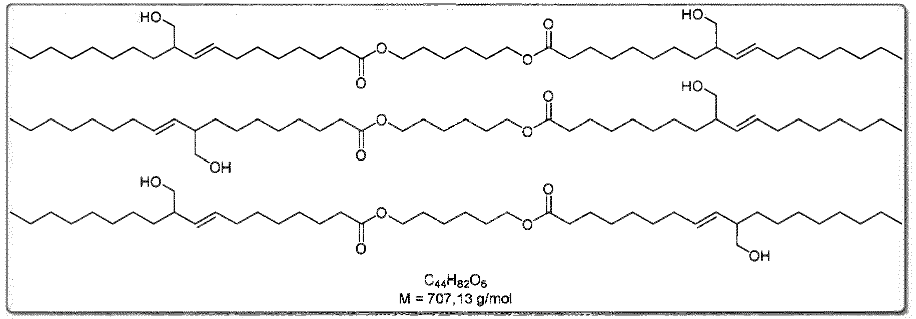

Die Synthese erfolgte nach AW 2. (*Z*)-Hexan-1,6-diyldioleat (1,73 g, 2,68 mmol) und Paraformaldehyd (373 mg, 12,4 mmol) wurden unter Zugabe von EtAlCl₂ (19 ml, 19 mmol) umgesetzt. Aufarbeitung und Säulenchromatographie (Cyclohexan/Ethylacetat 15:1, v/v) lieferte das gewünschte Produkt als farbloses Öl.

**Ausbeute:** 353 mg, 19%.

**¹H-NMR** (500 MHz, CDCl₃): δ [ppm] = 5.51 (m, 2H, C*H*=CHCH), 5.13 (m, 2H, CH=C*H*CH), 4.06 (m, 4H, COOC*H₂*), 3.53 (m, 2H, C*H₂*OH) 3.32 (m, 2H, C*H₂*OH), 2.29 (t, 4H, C*H₂*COO), 2.14 (m, 2H, CH=CHC*H*), 2.02 (m, 4H, C*H₂*CH=CH), 1.63 (m, 8H, C*H₂*CH₂COO, OCH₂C*H*), 1.32 (m, 42H), 0.88 (t, 6H, C*H*₃).

**¹³C-NMR** (125 MHz, CDCl₃): δ [ppm] = 134.29, 133.92, 131.62, 131.34, 66.13, 64.32, 46.10, 34.50, 34.48, 32.83, 32.73, 32.04, 32.01, 31.28, 31.24, 29.82, 29.69, 29.64, 29.46, 29.34, 29.29, 29.26, 29.11, 28.91, 28.71, 27.25, 27.19, 25.78, 25.13, 25.09, 22.81, 14.26.

**HRMS** (ESI): berechnet für C₄₄H₈₂O₆Na⁺: 729,6004; gefunden: 729,6014.

### Allgemeine Versuchsvorschrift 5 (AW5): Herstellung höhermolekularer Fettsäureester unter Einsatz der Säurechloride

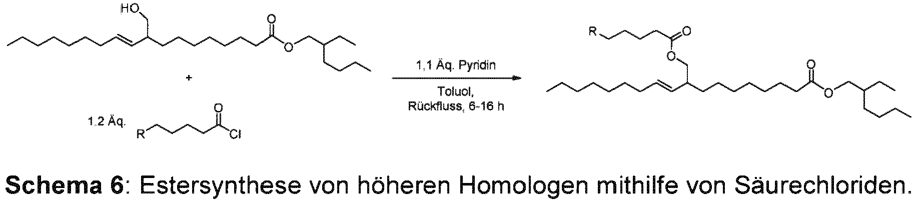

Die Säurechloride wurden kommerziell erworben oder gemäß folgender Vorschrift dargestellt: Der in Toluol gelösten Carbonsäure (1,0 Äq.) wurde unter Rühren bei 0 °C langsam Thionylchlorid (15 Aq.) zugetropft. Nach 16 h Erhitzen unter Rückfluss wurde das überschüssige Thionylchlorid sowie das Lösungsmittel mittels Mikrodestillation entfernt, um das Säurechlorid zu erhalten. Dieses wurde unmittelbar verwendet.

Die Fettsäurekomponente mit freier Alkoholfunktion (1,0 Äq.) wurde in Toluol mit Pyridin (1,1 Äq.) vorgelegt und unter Rühren bei Eiskühlung langsam mit dem Säurechlorid (1,2 Äq.) versetzt. Das Gemisch wurde innerhalb 15 min auf Raumtemperatur gebracht und anschließend für 6-16 h unter Rückfluss erhitzt. Nach Phasentrennung wurde die wässrige Phase mit Essigsäure angesäuert und noch zweimal mit Ethylacetat extrahiert. Die vereinigten Extrakte wurden über Magnesiumsulfat getrocknet und bei vermindertem Druck zur Trockne eingeengt. Säulenchromatographie lieferte die gewünschten Produkte.

### Beispiel 17

### Herstellung von einem Gemisch aus 9-(((12-(Hexanoyloxy)-octadecanoyl)-oxy)methyl)octadecansäure-(2'-ethylhexyl)ester und 10-(((12-(Hexanoyloxy)-octadecanoyl)-oxy)methyl)octadecansäure-(2'-ethylhexyl)ester

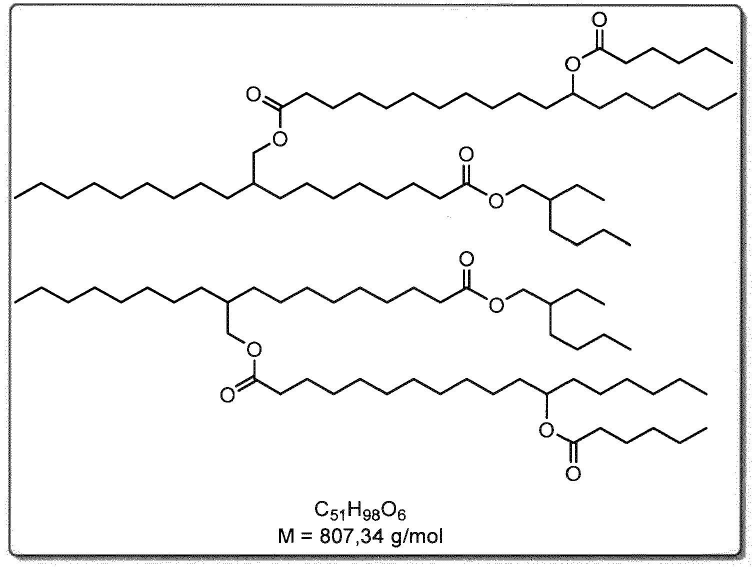

Die Synthese erfolgte nach AW 5. Ein Gemisch aus 9-(((12-Hydroxyoctadecanoyl)-oxy)methyl)octadecansäure-(2'-ethylhexyl)ester und 10-(((12-Hydroxyoctadecanoyl)-oxy)methyl)octadecansäure-(2'-ethylhexyl)ester (2,04 g, 2,87 mmol) wurde mit Pyridin (0,25 mL, 3,16 mmol) und Hexansäurechlorid (0,5 mL, 3,58 mmol) umgesetzt. Aufarbeitung und Säulenchromatographie über eine C18-RP-Säule (Acetonitril) lieferte das gewünschte Produkt als farbloses Öl.

**Ausbeute:** 8 mg, 2%. 4.86 (s, 1H), 4.01 - 3.91 (m, 8H), 2.27 (s, 5H), 1.76 - 1.15 (m, 64H), 0.89 (s, 9H).

**¹H-NMR** (500 MHz, CDCl₃): δ [ppm] = 4,86 (m, 1H, COOC*H*), 4.01-3.91 (m, 4H, COOC*H₂*); 2.27 (m, 6H, C*H₂*COO); 1.76-1.15 (m, 72H); 0.88 (m, 15H, C*H₃*).

**HRMS** (ESI): berechnet für C₅₁H₉₈O₆Na⁺: 829,7256; gefunden: 829,7261.

### Beispiel 18

### Herstellung von einem Gemisch 6-(((E)-9-((stearoyloxy)methyl)octadec-10-enoyl)oxy)hexyl-(E)-10-((stearoyloxy)methyl)octadec-8-ensäureester, hexane-1,6-diyl (10E,10'E)-bis(9-((stearoyloxy)methyl)octadec-10-ensäureester) und hexane-1,6-diyl (8E,8'E)-bis(10-((stearoyloxy)methyl)octadec-8-ensäureester)

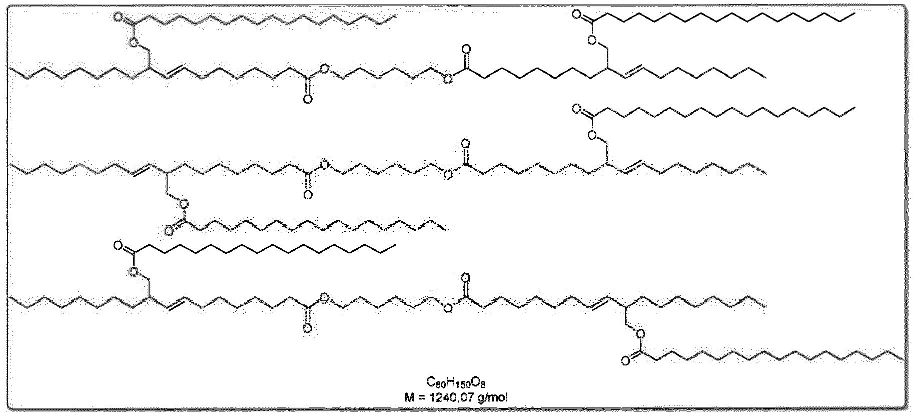

Die Synthese erfolgte nach AW 5. Ein Gemisch aus 6-(((*E*)-9-(hydroxymethyl)octadec-10-enoyl)oxy)hexyl-(*E*)-10-(hydroxymethyl)octadec-8-ensäureester, hexane-1,6-diyl (10*E*,10'*E*)-bis(9-(hydroxymethyl)octadec-10-ensäureester) und hexane-1,6-diyl (8*E*,8'*E*)-bis(10-(hydroxymethyl)octadec-8-ensäureester) (181 mg, 0,26 mmol) wurde mit Pyridin (0,25 µL, 0,28 mmol) und Stearinsäurechlorid (95,6 mg, 0,31 mmol) umgesetzt. Aufarbeitung und präparative Dünnschichtchromatographie lieferte das gewünschte Produkt als farbloses Öl.

**Ausbeute:** 20 mg, 3%.

**¹H-NMR** (500 MHz, CDCl₃): δ [ppm] = 5.43 (m, 2H, CH=C*H*), 5.15 (m, 2H, C*H*=CH), 4.04 (m, 4H, COOC*H*₂), 3.94 (t, 4H, COOC*H*₂-(CH₂)₄-C*H*₂OOC), 2.28 (m, 4H, C*H*₂COO), 1.98 (m, 4H, C*H*₂CH₂), 1.61 (m, 10H, C*H*₂CH₂COO + OCH₂C*H*), 1.45-1.11 (m, 108H), 0.88 (t, 12H, C*H₃*).

**HRMS** (ESI): berechnet für C₈₀H₁₅₀O₈Na⁺: 1261,1223; gefunden: 1262,1246.

### Beispiel 19

### Herstellung von einem Gemisch aus (E)-9-((stearoyloxy)methyl)octadec-10-ensäure und (E)-10-((stearoyloxy)methyl)octadec-8-ensäure

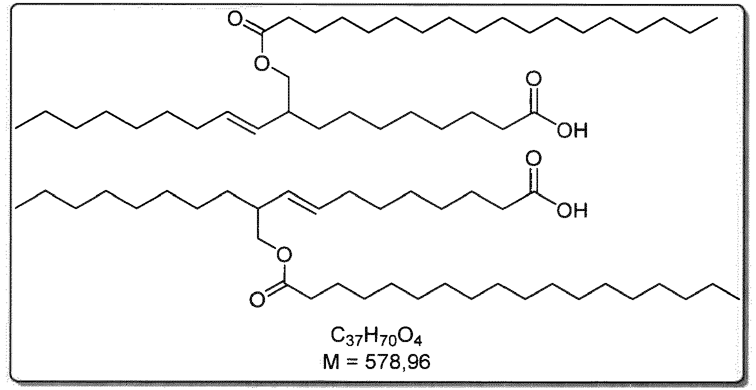

Die Synthese erfolgte nach AW 5. Ein Gemisch aus E-9-(Hydroxymethyl)octadec-10-ensäure und *E*-10-(Hydroxymethyl)octadec-8-ensäure (428 g, 1,3 mmol) wurde mit Pyridin (0,13 mL, 1,65 mmol) und Stearinsäurechlorid (aus Stearinsäure: 467 mg, 1,64 mmol) umgesetzt. Aufarbeitung und Filtration über Kieselgel (Cyclohexan:Ethylacetat 15:1 v/v) lieferte das gewünschte Produkt als farbloses Wachs.

**Ausbeute:** 716 mg, 92%.

**¹H-NMR** (500 MHz, CDCl₃): δ [ppm] = 5.42 (m, 1H, C*H*=CH-CH), 5.21 (m, 1H, CH=C*H*-CH), 3.94 (m, 2H, CH-C*H*₂-O), 2.34 (m, 2H, C*H*₂COOH), 2.28 (m, 2H, C*H*₂-COOCH₂), 2.04-1.93 (m, 2H, C*H*₂-CH=CH), 1.61 (m, 5H. CH=CH-C*H*. C*H*₂-CH₂-COO), 1.25 (s, 47H), 0.88 (t, ³*J* = 6.9 Hz, 6H, C*H₃*).

**¹³C-NMR** (125 MHz, CDCl₃): δ [ppm] = 179.15, 179.07, 134.33, 133.88, 131.64, 131.30, 66.15, 66.10, 46.06, 34.01, 34.01, 32.82, 32.68, 32.03, 32.01, 31.27, 31.21, 29.82, 29.69, 29.68, 29.57, 29.45, 29.37, 29.29, 29.28, 29.26, 29.16, 28.99, 28.79, 27.24, 27.15, 27.07, 24.80, 24.75, 22.82, 22.81, 14.25.

**HRMS** (ESI): berechnet für C₃₇H₇₀O₄Na⁺: 601,5166; gefunden: 601,5158.

| | | |
|---|---|---|
| **EA:** | berechnet für C₃₇H₇₀O₄ gefunden: | C: 76,76%, H: 12,19%, C: 76,71%, H: 12,42 % |

### Beispiel 20

Herstellung von einem Gemisch aus (*E*)-9-((((*E*)-9-((stearoyloxy)methyl)octadec-10-enoyl)oxy)methyl)octadec-10-ensäure-(2'-ethylhexyl)ester,
(*E*)-9-((((*E*)-9-((stearoyloxy)methyl)octadec-10-enoyl)oxy)methyl)octadec-8-ensäu re-(2'-ethylhexyl)ester,
(*E*)-10-((((*E*)-9-((stearoyloxy)methyl)octadec-10-enoyl)oxy)methyl)octadec-10-ensäure-(2'-ethylhexyl)ester und
(*E*)-10-((((*E*)-9-((stearoyloxy)methyl)octadec-10-enoyl)oxy)methyl)octadec-8-ensäure-(2'-ethylhexyl)ester

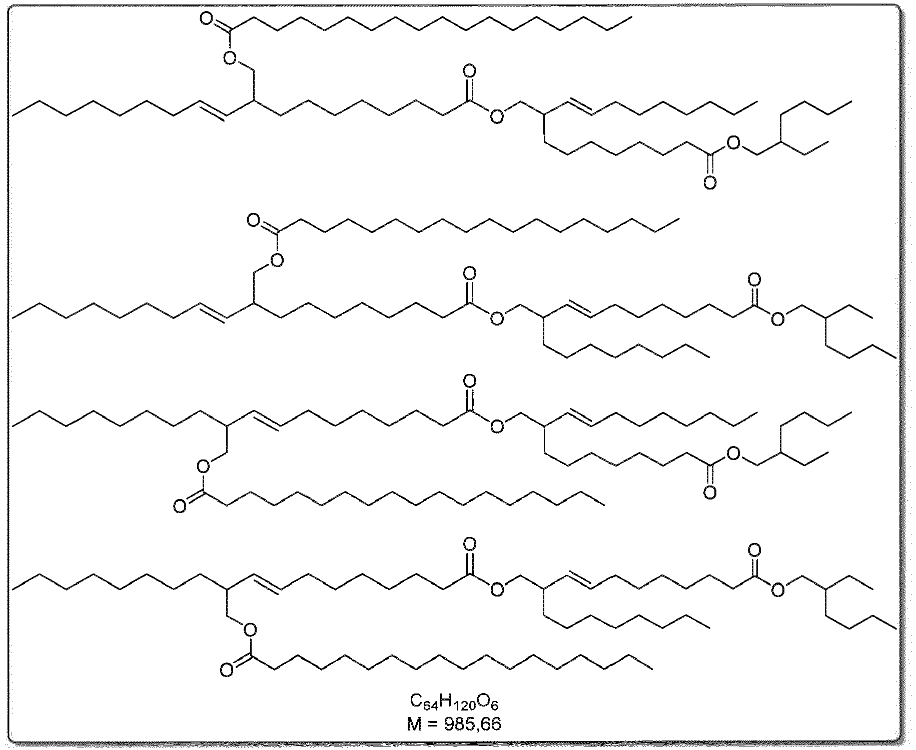

Ein Gemisch aus (*E*)-9-((stearoyloxy)methyl)octadec-10-ensäure und (*E*)-10-((stearoyloxy)methyl)octadec-8-ensäure (271 mg, 0,47 mmol) wurde mit einem Gemisch aus *E*-9-(Hydroxymethyl)octadec-10-ensäure-(2'-ethylhexyl)ester und *E*-10-(Hydroxymethyl)octadec-8-ensäure-(2'-ethylhexyl)ester (188 mg, 0,44 mmol) in Toluol vorgelegt und mit p-Toluolsulfonsäure (84 mg, 0,44 mmol) und Molekularsieb 4Å (62 mg, 121,2 mg/mmol) versetzt. Nach 2 h Rühren bei 40 °C wurde Eis hinzugegeben. Nach Phasentrennung wurde die wässrige Phase dreimal mit Ethylacetat (1:1 v/v) extrahiert. Die vereinigten organischen Phasen wurden mit gesättigter wässriger Natriumhydrogencarbonatlösung und anschließend mit gesättigter wässriger Natriumchloridlösung gewaschen, über Magnesiumsulfat getrocknet und unter vermindertem Druck zur Trockne eingeengt. Säulenchromatographie (Cyclohexan: Ethylacetat 20:1 v/v) lieferte das gewünschte Produkt als farbloses Öl.

**Ausbeute:** 14 mg, 3%.

**¹H-NMR** (500 MHz, CDCl₃): δ [ppm] = 5.42 (m, 2H, C*H*=CH-CH), 5.21 (m, 2H, CH=C*H*-CH), 3.98 (m, 2H, CH-C*H*₂-O-Ethylhexyl), 3.96-3.87 (m, 4H, CH-C*H*₂-O), 2.28(m, 6H, C*H*₂-COOCH₂), 1.98 (m, 4H, C*H*₂-CH=CH), 1.68-1.50 (m, 8H. CH=CH-C*H*. C*H*₂-CH₂-COO), 1.43-1.15 (s, 77H), 0.88 (t, ³*J* = 4.7 Hz, 15H, C*H₃*).

**¹³C-NMR** (125 MHz, CDCl₃): δ [ppm] = 174.21, 174.17, 174.05, 174.00, 173.96, 132.77, 132.47, 130.76, 130.75, 130.59, 67.74, 67.72, 66.79, 42.25, 42.20, 38.90, 34.59, 34.58, 34.55, 32.77, 32.72, 32.70, 32.08, 32.04, 31.61, 30.58, 29.86, 29.83, 29.82, 29.80, 29.69, 29.66, 29.52, 29.47, 29.46, 29.42, 29.40, 29.35, 29.34, 29.22, 29.21, 29.19, 29.17, 29.08, 28.95, 28.91, 27.05, 27.03, 25.20, 25.19, 25.16, 25.14, 23.95, 23.14, 22.85, 22.83, 14.27, 14.21, 11.15.

**HRMS** (ESI): berechnet für C₆₄H₁₂₀O₆Na⁺: 1007,8984; gefunden: 1007,8977.

| | | |
|---|---|---|
| **EA:** | berechnet für C₃₇H₇₀O₄ gefunden: | C: 77,99%, H: 12,27%, C: 78,17%, H: 12,30 % |

## Patentansprüche

1. Mischung aus mindestens 2 Esterverbindungen der allgemeinen Formel (la) 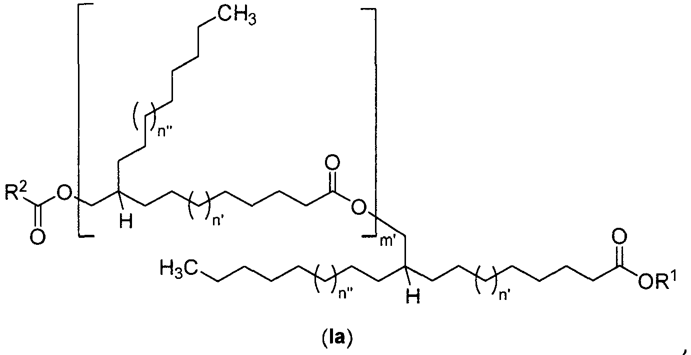 aufweisen, in der
n' und n" entweder 1 und 2 oder 2 und 1 ist,
m' 1 bis 5 ist,
der Rest R¹ ausgewählt wird aus der Gruppe bestehend aus Wasserstoff, verzweigten oder unverzweigten C₁- bis C₆₀-Alkylresten, verzweigten oder unverzweigten C₂- bis C₆₀-Alkenylresten, C₇- bis C₆₀-Arylalkylresten, C₁- bis C₆₀-Heteroarylalkylresten und/oder C₆- bis C₆₀-Arylreste,
der Rest R² ausgewählt wird aus der Gruppe bestehend aus verzweigten oder unverzweigten C₁- bis C₆₀-Alkylresten, verzweigten oder unverzweigten C₂- bis C₆₀-Alkenylresten, C₇- bis C₆₀-Arylalkylresten und/oder C₆- bis C₆₀-Heteroarylalkylresten und/oder cyclisch gesättigten und oder ungesättigten C₅- bis C₆₀-Alkylresten, wobei diese unsubstituiert oder einfach oder mehrfach mit wenigstens einem Substituenten ausgewählt aus der Gruppe aus OH substituiert sind.

2. Mischung aus mindestens 2 Esterverbindungen der allgemeinen Formel **(Ib)** 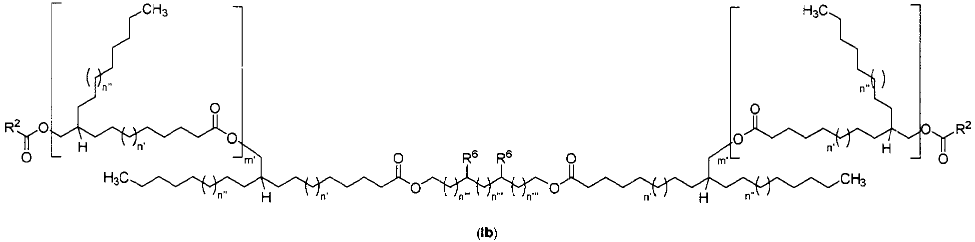 aufweisen, in der
n' und n" entweder 1 und 2 oder 2 und 1, n‴ gleich 0 bis 10 sind,
m' gleich 0 bis 5 ist, vorzugsweise 1 bis 5,
der Rest R² ausgewählt wird aus der Gruppe bestehend aus verzweigten oder unverzweigten C₁- bis C₆₀-Alkylresten, verzweigten oder unverzweigten C₁- bis C₆₀-Alkenylresten, C₇- bis C₆₀-Arylalkylresten und/oder C₆- bis C₆₀-Heteroarylalkylresten und/oder cyclisch gesättigten und oder ungesättigten C₅- bis C₆₀-Alkylresten, wobei diese unsubstituiert oder einfach oder mehrfach mit wenigstens einem Substituenten ausgewählt aus der Gruppe aus OH substituiert sind,
Rest R⁶ ausgewählt wird aus der Gruppe bestehend aus verzweigten oder unverzweigten C₁- bis C₆₀-Alkylresten sowie verzweigten oder unverzweigten C₂- bis C₆₀-Alkenylresten.

3. Mischung aus mindestens 2 Esterverbindungen der allgemeinen Formel **(Ic)** 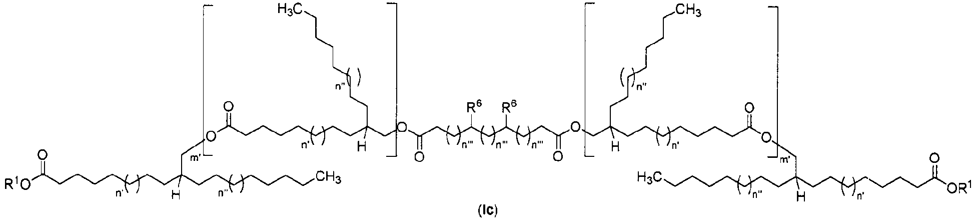 aufweisen, in der
n' und n" entweder 1 und 2 oder 2 und 1, n‴ gleich 0 bis 10 sind,
m' gleich 0 bis 5,
der Rest R¹ ausgewählt wird aus der Gruppe bestehend aus Wasserstoff, verzweigten oder unverzweigten C₁- bis C₆₀-Alkylresten, verzweigten oder unverzweigten C₁- bis C₆₀-Alkenylresten, C₇- bis C₆₀-Arylalkylresten, C₁- bis C₆₀-Heteroarylalkylresten und/oder C₆- bis C₆₀-Arylreste,
der Rest R⁶ ausgewählt wird aus der Gruppe bestehend aus verzweigten oder unverzweigten C₁- bis C₆₀-Alkylresten sowie verzweigten oder unverzweigten C₂- bis C₆₀-Alkenylresten.

4. Mischung aus Esterverbindungen nach einem der vorherigen Ansprüche 1 bis 3, **dadurch gekennzeichnet, dass** jeweils zwei dieser Verbindungen Regioisomere zueinander darstellen.

## Claims

1. Mixture comprising at least two ester compounds of the general formula **(Ia)** 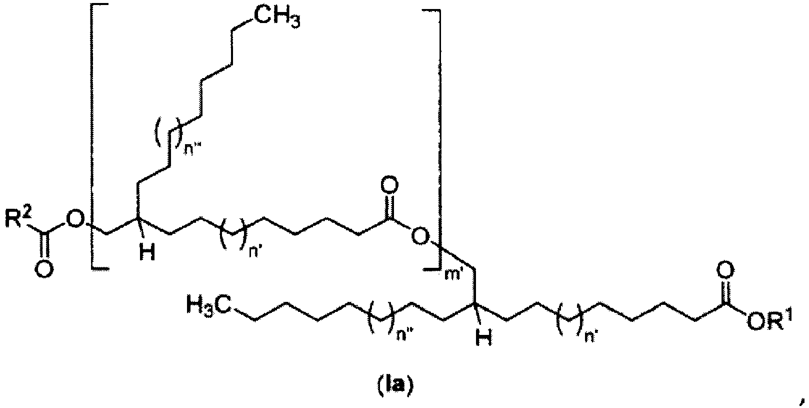 wherein
n' and n" are either 1 and 2 or 2 and 1,
m' is 1 to 5,
the remainder R¹ is selected from the group consisting of hydrogen, branched or unbranched C₁- to C₆₀ alkyl residues, branched or unbranched C₂- to C₆₀ alkenyl residues, C₇- to C₆₀ arylalkyl residues, C₁- to C₆₀ heteroaryalkyl residues, and/or C₆- to C₆₀ aryl residues,
the remainder R² is selected from the group consisting of branched or unbranched C₁- to C₆₀ alkyl residues, branched or unbranched C₂- to C₆₀ alkenyl residues, C₇- to C₆₀ arylalkyl residues, and/or C₆- to C₆₀ heteroarylalkyl residues, and/or cyclic saturated or unsaturated C₅- to C₆₀ alkyl residues, wherein these are unsubstituted or substituted singly or multiple times with at least one substitute selected from the group from OH.

2. Mixture comprising at least two ester compounds of the general formula **(Ib)** 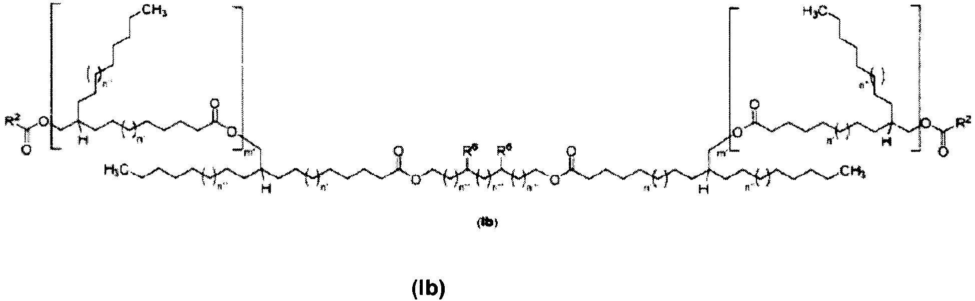 wherein
n' and n" are either 1 and 2 or 2 and 1, n‴ is equal to 0 to 10,
m' is equal to 0 to 5, preferably 1 to 5,
the remainder R² is selected from the group consisting of branched or unbranched C₁- to C₆₀ alkyl residues, branched or unbranched C₁- to C₆₀ alkenyl residues, C₇- to C₆₀ arylalkyl residues, and/or C₆- to C₆₀ heteroarylalkyl residues and/or cyclic saturated or unsaturated C₅- to C₆₀ alkyl residues, wherein these are unsubstituted or substituted singly or multiple times with at least one substitute selected from the group from OH,
the remainder R⁶ is selected from the group consisting of branched or unbranched C₁- to C₆₀ alkyl residues and branched or unbranched C₂- to C₆₀ alkenyl residues.

3. Mixture comprising at least two ester compounds of the general formula **(Ic)** 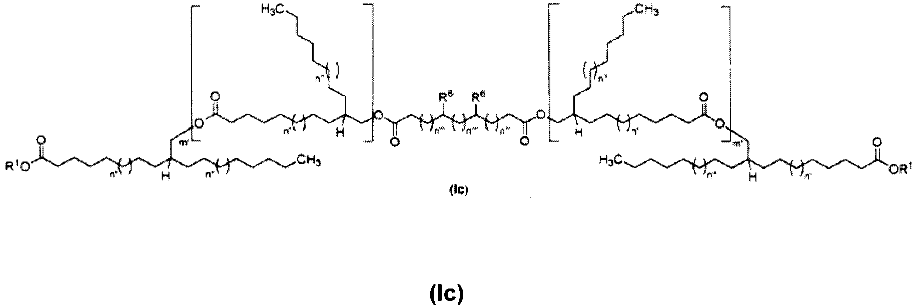 wherein
n' and n" are either 1 and 2 or 2 and 1, n‴ is equal to 0 to 10,
m' is equal to 0 to 5,
the remainder R¹ is selected from the group consisting of hydrogen, branched or unbranched C₁- to C₆₀ alkyl residues, branched or unbranched C₁- to C₆₀ alkenyl residues, C₇- to C₆₀ arylalkyl residues, C₁- to C₆₀ heteroarylalkyl residues, and/or C₂- to C₆₀ alkyl residues,
the remainder R⁶ is selected from the group consisting of branched or unbranched C₁- to C₆₀ alkyl residues, and branched or unbranched C₂- to C₆₀ alkenyl residues,

4. Mixture of ester compounds according to any one of the preceding claims 1 to 3, **characterized in that** in each case two of these compounds represent regiosomers in relation to one another.

## Revendications

1. Mélange d'au moins deux composés d'esters présentant la formule générale (la) 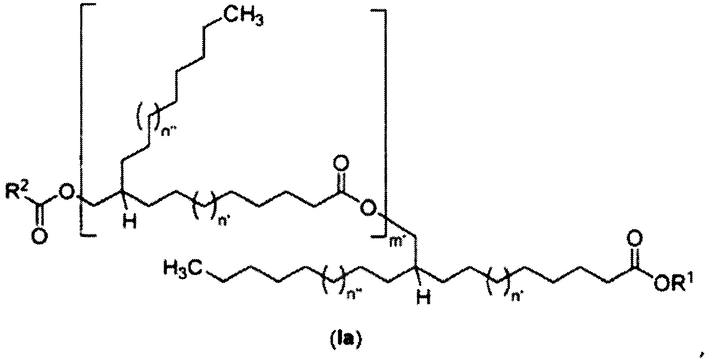 où
n'et n" sont soit 1 et 2, soit 2 et 1,
m' est 1 à 5,
le reste R¹ est sélectionné parmi le groupe constitué d'hydrogène, de restes alkyle en C₁- C₆₀ ramifiés ou non ramifiés, de restes alcényle en C₂- à C₆₀ ramifiés ou non ramifiés, de restes arylalkyle en C₇ à C₆₀, de restes d'hétéroarylalkyle en C₁ à C₆₀ et/ou de restes aryle en C₆ à C₆₀,
le reste R² est sélectionné parmi le groupe constitué de restes alkyle en C₇ à C₆₀ ramifiés ou non ramifiés, de restes alcényle en C₂ à C₆₀ ramifiés ou non ramifiés, de restes arylalkyle en C₇ à C₆₀ et/ou des restes d'hétéroarylalkyle en C₆ à C₆₀ et/ou de restes alkyle en C₅ à C₆₀ saturés et ou non saturés cycliquement, ceux-ci étant non substitués ou substitués une ou plusieurs fois par au moins un substituant sélectionné parmi le groupe OH.

2. Mélange d'au moins deux composés d'esters présentant la formule générale **(Ib)** 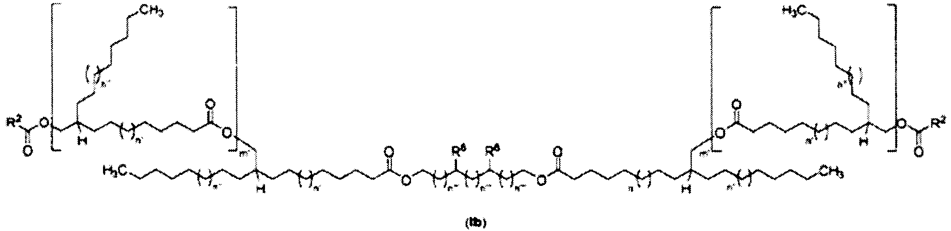 où
n'et n" sont soit 1 et 2 ou 2 et 1, n" étant égal à 0 à 10,
m' est égal à 0 à 5, de préférence de 1 à 5,
le reste R² est sélectionné parmi le groupe constitué de restes alkyle en C₁ à C₆₀ ramifiés ou non ramifiés, de restes d'alcényle en C₁ à C₆₀ ramifiés ou non ramifiés, de restes alcényle en C₁ à C₆₀ ramifiés ou non ramifiés, de restes arylalkyle en C₇ à C₆₀ et/ou de restes hétéroarylalkyle en C₆ à C₆₀ et/ou de restes alkyle en C₅ à C₆₀ saturés ou non saturés cycliquement, ceux-ci étant non substitués ou substitués une ou plusieurs fois par au moins un substituant sélectionné parmi le groupe OH,
le reste R⁶ est sélectionné parmi le groupe constitué de restes alkyle en C₁ à C₆₀ ramifiés ou non ramifiés, ainsi que de restes alcényle en C₂ à C₆₀ ramifiés ou non ramifiés.

3. Mélange d'au moins deux composés d'esters présentant la formule générale (Ic), 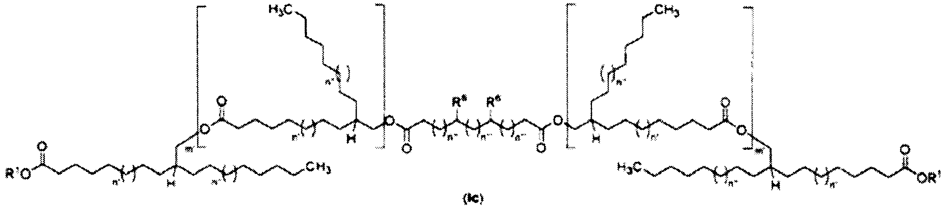 où
n'et n" sont soit 1 et 2 , soit 2 et 1, n‴ est égal à 0 à 10,
m' est égal à 0 à 5,
le reste R¹ est sélectionné parmi le groupe constitué d'hydrogène, de restes alkyle en C₁ à C₆₀ ramifiés ou non ramifiés, de restes alcényle en C₁ à C₆₀ ramifiés ou non ramifiés, de restes arylalkyle en C₇ à C₆₀, de restes hétéroarylalkyle en C₁ à C₆₀ et/ou de restes aryle en C₆ à C₆₀,
le reste R⁶ est sélectionné parmi le groupe constitué de restes alkyle en C₁ à C₆₀ ramifiés ou non ramifiés, ainsi que de restes alcényle en C₂ à C₆₀ ramifiés ou non ramifiés.

4. Mélange de composés d'esters suivant une des revendications précédentes 1 à 3, **caractérisé en ce que** respectivement deux de ces composés représentent des régioisomères l'une par rapport à l'autre.
